# EUROPÄISCHE PATENTSCHRIFT

(11) **EP 4 312 902 B1**
(45) Veröffentlichungstag und Bekanntmachung des Hinweises auf die Patenterteilung: **12.02.2025**
(21) Anmeldenummer: 22717567.6
(22) Anmeldetag: 22.03.2022
(51) Int. Cl.: A61F 5/01

(54) **FUSSORTHESE MIT ZEHENSEGMENT IN FORM EINES BÜGELS ZUR KORREKTUR VON FUSSFEHLSTELLUNGEN**
FOOT ORTHOSIS WITH BRACKET-SHAPED TOE SEGMENT FOR CORRECTING MALPOSITIONS OF THE FOOT
ORTHÈSE DE PIED AVEC SEGMENT D'ORTEIL EN FORME DE SUPPORT POUR CORRIGER LES MALPOSITIONS DU PIED

(30) Priorität: 22.03.2021 DE 102021107082
(43) Veröffentlichungstag der Anmeldung: 07.02.2024
(73) Patentinhaber: Elsa Vermögens- und Beteiligungs AG, 82031 Grünwald (DE)
(72) Erfinder: BRASS, Manfred, 82031 Grünwald (DE); OSTENRIEDER, Jörg, 82067 Ebenhausen (DE)
(74) Vertreter: Bulat, Branimir
(86) Internationale Anmeldenummer: PCT/EP2022/057523
(87) Internationale Veröffentlichungsnummer: WO 2022/200368

(56) Entgegenhaltungen:
- DE-U1- 8 529 083
- US-A- 1 183 062
- US-A- 2 958 324
- US-A1- 2012 310 131
- US-A1- 2014 350 447

## Beschreibung

### Technisches Gebiet

Die vorliegende Erfindung betrifft eine Fußorthese zur Korrektur von Fehlstellungen, insbesondere zur Behandlung von Hallux valgus.

### Stand der Technik

Pathologische Fehlstellungen im Mittelfuß- und Vorfußbereich eines Patienten können unterschiedliche Ursachen haben, wie beispielsweise genetische Veranlagung, das Tragen von falschem Schuhwerk, insbesondere von zu engen oder hochhackigen Schuhen, oder eine Abflachung des Längs- und Quergewölbes infolge einer Instabilität des Bindegewebes im Mittelfußbereich. Insbesondere die als Hallux valgus bezeichnete Fehlstellung der Großzehe im Großzehengrundgelenk, auch als Valgusstellung bezeichnet, kommt hierbei aufgrund der stetig wachsenden Fallzahlen mehr Bedeutung zu.

Hallux valgus entsteht, indem die Großzehe im Großzehengrundgelenk durch Muskelzug in die fußinnenseitige Richtung gezogen wird. Dabei tritt der erste Mittelfußknochen als ballenförmige Ausbuchtung am Zehengrundgelenk fußinnenseitig hervor, was als Pseudoexostose bezeichnet wird. Zudem geht mit dem Hallux valgus häufig eine Änderung der Länge und der Zugrichtung der Sehnen einher, wodurch sich die Fehlstellung im Laufe der Zeit weiter verstärken kann. Dabei entwickelt sich eine Arthrose des Großzehengrundgelenks, die im fortgeschrittenen Stadium chirurgisch behandelt werden muss.

Um den Krankheitsprozess aufzuhalten oder diesem entgegenzuwirken, sind neben chirurgischen Eingriffen auch die Anwendung konservativer Therapiemethoden bekannt. Beispielsweise ist die Verwendung von Tapeverbänden oder Orthesen bekannt zur Behandlung des Fußes in einer Ruhestellung. Aufgrund der verlangten Ruhestellung des Fußes während der Therapie werden diese vorwiegend nachts eingesetzt.

Weiterhin sind Orthesen bekannt, die in einem am Fuß befestigten Zustand einer geschienten Großzehe einen Bewegungsfreiheitsgrad entlang der Flexion-Extension-Bewegungsrichtung einräumen. DE 102 40 121 B4 offenbart beispielsweise eine orthopädische Vorrichtung in Form einer Gelenkbiegeschiene, die um eine Flexions-Extensions-Bewegungsachse der zu korrigierenden Zehe gelenkig ausgebildet ist. Hierzu ist die Gelenkbiegeschiene mit einer an der Fußinnenseite anliegenden Gelenkeinrichtung versehen, von der sich zwei Biegeschenkel entlang der Fußinnenseite erstrecken. Um die Gelenkbiegeschiene am Fuß zu befestigen, ist ein erster Biegeschenkel über eine erste Bandage an der Zehe und ein zweiter Biegeschenkel über eine zweite Bandage am Mittelfuß befestigt.

US 2,958,324 A offenbart eine Vorrichtung zur Behandlung von Zehenfehlstellungen. Die Vorrichtung umfasst einen U-förmigen Ballenabschnitt, dessen ober- und unterseitig am Fuß anliegende Schenkel mit einem Zehenbügel gelenkig verbunden sind. Weiterer Stand der Technik ist aus der DE 85 29 083 U1, US 1,183,062 A, US 2014/0350447 A1 und US 2012/310131 A1 bekannt.

### Darstellung der Erfindung

Ausgehend von dem bekannten Stand der Technik ist es eine Aufgabe der vorliegenden Erfindung, eine verbesserte Fußorthese zur Korrektur von Fehlstellungen, insbesondere zur Behandlung von Hallux valgus, bereitzustellen, die insbesondere eine effektive therapeutische Behandlung sicherstellt und gleichzeitig aufwandsreduziert herstellbar und kompakt ausgestaltet ist.

Die Aufgabe wird durch eine Fußorthese mit den Merkmalen des unabhängigen Anspruchs gelöst. Vorteilhafte Weiterbildungen ergeben sich aus den Unteransprüchen sowie der vorliegenden Beschreibung und den Figuren.

Entsprechend wird eine Fußorthese zur Korrektur von Fehlstellungen eines Fußes, insbesondere zur Behandlung oder Vorbeugung von Hallux valgus, bereitgestellt. Die Fußorthese umfasst ein an einer Zehe des zu behandelnden Fußes befestigbares Zehensegment und ein im Bereich eines Zehengrundgelenks des zu behandelnden Fußes anzuordnendes Ballensegment, die mittels einer Gelenkeinheit relativ zueinander verschwenkbar gekoppelt sind. Die Fußorthese ist dazu eingerichtet und derart ausgebildet, in einem am Fuß ordnungsgemäß befestigten Zustand über das Zehensegment eine erste Korrekturkraft auf die Zehe auszuüben und über das Ballensegment eine zu der ersten Korrekturkraft entgegengesetzte zweite Korrekturkraft auf das Zehengrundgelenk auszuüben. Die Gelenkeinheit ist derart ausgebildet, dass das Zehensegment und das Ballensegment relativ zueinander um eine Schwenkachse verschwenkbar sind, die im Wesentlichen parallel zu einer Flexion-Extension-Gelenkachse des Zehengrundgelenks ist. Das Zehensegment ist in Form eines Bügels ausgebildet, der in dem am Fuß befestigten Zustand die Zehe zumindest teilweise umgreift. Das Zehensegment umfasst einen Zehenstützabschnitt, der in dem am Fuß befestigten Zustand der Fußorthese an einer lateralen Seite der Zehe anliegt.

Zur Interaktion mit der Zehe des zu behandelnden Fußes, d.h. zum Ausüben der ersten Korrekturkraft auf die Zehe, ist die Fußorthese mit dem als Bügel ausgebildeten und die Zehe umgreifenden Zehensegment ausgestattet. Im Rahmen der vorliegenden Erfindung wurde erkannt, dass indem das Zehensegment als ein die Zehe zumindest teilweise umgreifender Bügel ausgestaltet ist, eine für die vorgesehene Anwendung der Fußorthese kraftfluss- und belastungsoptimierte strukturelle Ausgestaltung bereitgestellt werden kann. Denn indem das bügelartige Zehensegment die Zehe zumindest teilweise umgreift, wird ermöglicht, dass durch die sich so ergebende Geometrie des Zehensegments die Steifigkeit des Bauteils, insbesondere dessen Flächenträgheitsmoment, im Hinblick auf die in dem Zehensegment übertragenen, die erste Korrekturkraft induzierenden Biegekräfte zumindest abschnittsweise erhöht wird. Auf diese Weise kann das Zehensegment zumindest abschnittsweise schmaler und/oder dünner ausgestaltet sein und entsprechend zu einer kompakten Bauweise der Fußorthese beitragen. Das Tragen der Fußorthese in üblichem Schuhwerk kann so für den Patienten gegenüber bekannten Vorrichtungen, bei denen sich Biegeschenkel entlang der Zehe an der Fußinnenseite erstrecken, deutlich angenehmer sein. Diese Ausgestaltung der vorgeschlagenen Lösung ermöglicht weiterhin, dass auf Bandagen zum Befestigen der Fußorthese an dem Fuß verzichtet werden kann. Entsprechend kann die vorgeschlagene Fußorthese weniger unterschiedliche Komponenten umfassen und daher kostengünstiger in der Herstellung sein.

Die vorgeschlagene Fußorthese ist dafür vorgesehen, eine pathologische Fehlstellung eines Fußes, insbesondere einer Zehe und/oder eines Zehengrundgelenks, zu behandeln, entgegenzuwirken und/oder vorzubeugen. Insbesondere kann die Fußorthese zur Vorbeugung oder Behandlung von Hallux valgus eingesetzt werden, ist aber nicht auf diese Anwendung beschränkt. Entsprechend ist die Fußorthese dafür vorgesehen, an einem Fuß eines Patienten befestigt zu werden und in dem am Fuß befestigten Zustand auf den Fuß, insbesondere auf die Zehe und/oder das Zehengrundgelenk, therapeutisch einzuwirken.

In der vorliegenden Offenbarung bezieht sich der Ausdruck "in einem/dem am Fuß ordnungsgemäß befestigten Zustand", vorliegend auch mit "in dem befestigten Zustand" abgekürzt, auf einen Zustand, in dem die Fußorthese an einem Fuß eines Patienten bestimmungsgemäß befestigt ist und entsprechend einen gewünschten therapeutischen Effekt zur Korrektur oder zur Vorbeugung von Fehlstellungen bewirkt. Die Fußorthese kann fußspezifisch für einen linken oder rechten Fuß eines Patienten ausgestaltet sein. Mit anderen Worten kann die Fußorthese entweder zur Verwendung an dem linken oder dem rechten Fuß des Patienten vorgesehen und ausgebildet sein. Eine für den linken Fuß vorgesehene Fußorthese kann spiegelsymmetrisch zu einer für den rechten Fuß eines Patienten vorgesehenen Fußorthese ausgebildet sein.

Die vorgeschlagene Fußorthese ist derart ausgestaltet, dass sie in dem befestigten Zustand Korrekturkräfte auf den Fuß ausübt. Unter dem Begriff "Korrekturkräfte" werden vorliegend solche Kräfte verstanden, die eine therapeutische Wirkung auf den zu behandelnden Fuß haben. Insbesondere bewirken die Korrekturkräfte, dass die von der Fehlstellung betroffenen Teile des Fußes in eine anatomisch korrekte oder beabsichtigte Position gelenkt werden, um einen gewünschten therapeutischen Effekt zu erzielen.

Die vorgeschlagene Fußorthese ist dazu eingerichtet, in dem am Fuß des Patienten befestigten Zustand wenigstens die erste und zweite Korrekturkraft mittels des Zehensegments und des Ballensegments auf den zu behandelnden Fuß auszuüben, insbesondere unmittelbar auszuüben. Durch diese Ausgestaltung unterscheidet sich die vorliegende Fußorthese wesentlich von bekannten Vorrichtungen, die ein Zehengrundgelenk und den diesem zugeordneten Zehenballen oder eine durch die Fehlstellung bewirkte pathologische Pseudoexostose vor äußerer Krafteinwirkung, insbesondere von durch die Vorrichtung auf den Fuß wirkenden Kräften, gewollt oder ungewollt abschirmen. Im Rahmen der vorliegenden Erfindung wurde erkannt, dass eine besonders effektive therapeutische Wirkung erzielt werden kann, indem die Fußorthese neben der auf die Zehe wirkenden ersten Korrekturkraft zusätzlich die auf das Zehengrundgelenk mittels des Ballensegments wirkende zweite Korrekturkraft ausübt. Das dadurch resultierende Zusammenspiel von auf den Fuß ausgeübten Korrekturkräften kann besonders vorteilhaft bei der Behandlung von Hallux valgus sein. Denn durch die vorgeschlagene Ausgestaltung der Fußorthese kann gleichzeitig eine therapeutische Wirkung auf die Valgusstellung der Zehe als auch auf die Varusstellung des Zehengrundgelenks erzielt werden. Somit können Symptomatik und Ursache der Fußfehlstellung gleichzeitig behandelt werden. Die auf den Fuß wirkenden Korrekturkräfte und damit einhergehenden therapeutischen Effekte werden nachstehend im Zusammenhang mit den damit in Verbindung stehenden Komponenten der Fußorthese genauer beschrieben.

Im Zusammenhang mit dem Zehensegment wird nachfolgend zur Vereinfachung allgemein auf eine Zehe des Fußes Bezug genommen. Hierbei handelt es sich insbesondere um die Großzehe des zu behandelnden Fußes. Die Fußorthese ist hierauf aber nicht beschränkt, sodass mit dem Ausdruck beispielsweise auch die Kleinzehe gemeint sein kann. Im Zusammenhang mit dem Ballensegment wird entsprechend zur Vereinfachung allgemein auf ein Zehengrundgelenk Bezug genommen, wobei insbesondere das Großzehengrundgelenk gemeint ist, die Fußorthese hierauf aber nicht beschränkt ist. Alternativ kann es sich beispielsweise auch um das Kleinzehengrundgelenk handeln.

In der vorliegenden Offenbarung wird zur Beschreibung der Fußorthese, insbesondere in Bezug auf den zu behandelnden Fuß, ein Bezugssystem verwendet, das auf die Mittellinie des Körpers eines Patienten ausgerichtet ist, wie in der Anatomie üblich. So können die Position und Richtung der einzelnen Komponenten der vorgeschlagenen Fußorthese in dem befestigten Zustand in Bezug auf den in der Fußorthese aufgenommenen Fuß angegeben werden. Dementsprechend bezieht sich der Begriff "medial" auf eine Richtung oder Seite der Fußorthese, die in Richtung einer medialen Ebene des Körpers des Trägers zeigt. In der Anatomie bezieht sich der Begriff "mediale Ebene", die auch als "Mittelsagittalebene" bezeichnet wird, im Allgemeinen auf eine anatomische Ebene, die den Körper in zwei symmetrische Teile teilt. Dementsprechend bedeutet der Begriff "in medialer Richtung" bei der Beschreibung einer Fußorthese eine Richtung, die von dem zu behandelnden Fuß des Patienten in Richtung seines anderen Fußes zeigt. In diesem Sinne bezieht sich der Begriff "lateral" auf eine Richtung oder Seite der Fußorthese, die von der medialen Ebene des Körpers des Trägers weg weist. Dementsprechend bedeutet der Begriff "in lateraler Richtung" bei der Beschreibung einer Fußorthese, welche an einem Fuß des Trägers befestigt ist, in einer Richtung, die von dem anderen Fuß des Trägers weg weist.

Die Fußorthese umfasst das Zehensegment, das an der Zehe des zu behandelnden Fußes befestigbar ist. Mit anderen Worten ist das Zehensegment dafür vorgesehen, an der Zehe des Fußes an einer vergebenen Position in Eingriff gebracht zu werden, um in dem am Fuß befestigten Zustand der Fußorthese eine kraftübertragende Kopplung zwischen der Zehe und dem Zehensegment bereitzustellen. Entsprechend wird das Zehensegment in dem am Fuß befestigten Zustand der Fußorthese in einer gewünschten Position an dem Fuß gehalten und insbesondere kraftschlüssig und/oder formschlüssig an der Zehe befestigt.

Das Zehensegment ist dafür vorgesehen, in dem am Fuß befestigten Zustand die erste Korrekturkraft auf die Zehe auszuüben. Die erste Korrekturkraft kann auf die Zehe in medialer Richtung wirken.

Wie vorangehend beschrieben, ist das Zehensegment in Form eines Bügels ausgebildet. Unter dem Begriff "Bügel" wird vorliegend und im Allgemeinen eine Komponente verstanden, die dazu eingerichtet und dafür vorgesehen ist, unterschiedliche Belastungen aufzunehmen und zu übertragen, wie zum Beispiel Längskräfte, Querkräfte, Scherkräfte, Biegekräfte, Biegemomente, Torsionsmomente, etc. So ist ein Bügel dafür vorgesehen, neben Zugkräften auch Druckkräfte entlang seiner Längsachse und Querkräfte quer zu seiner Erstreckungsachse, insbesondere seiner Längsachse, aufzunehmen und zu übertragen. Diese Eigenschaft unterscheidet einen Bügel wesentlich von einer Bandage, die zwar zur Übertragung von Zugkräften, aber nicht zur Übertragung von Druck- und/oder Querkräften vorgesehen ist.

Entsprechend kann das als Bügel ausgebildete Zehensegment dazu eingerichtet sein, in dem am Fuß befestigten Zustand Scherkräfte und/oder Biegekräfte, insbesondere in Richtung der ersten Korrekturkraft, aufzunehmen und zu übertragen, insbesondere zwischen der Gelenkeinheit und der zu behandelnden Zehe, um die erste Korrekturkraft auf die Zehe auszuüben.

Das als Bügel ausgebildete Zehensegment ist derart ausgestaltet, dass es in dem am Fuß befestigten Zustand die Zehe zumindest teilweise umgreift. Unter einem Umgreifen der Zehe wird vorliegend verstanden, dass das Zehensegment sich in dem am Fuß befestigten Zustand in Zehenumfangsrichtung entlang der Zehe erstreckt. Vorzugsweise erstreckt sich das Zehensegment um die Zehe über ein Bogenmaß von wenigstens 1/2 π rad um die Zehenlängsachse. Das bedeutet, dass sich das Zehensegment in Umfangsrichtung wenigstens entlang eines Viertels des Umfangs einer Zehe erstreckt. Das Zehensegment kann sich beispielsweise über ein Bogenmaß von im Wesentlichen einem π rad um die Zehenlängsachse erstrecken, sodass sich das Zehensegment von einer Seite der Zehe oder des Zehengrundgelenks zu der entgegengesetzten Seite der Zehe erstreckt.

Im Hinblick auf die geometrische Ausgestaltung ist das als Bügel ausgebildete Zehensegment vorzugsweise platten- und/oder schalenförmig ausgebildet. Beispielsweise kann das Zehensegment in seiner geometrischen Ausgestaltung riemen- oder bandförmig ausgebildet sein und insbesondere biegesteif sein. Entsprechend kann das Zehensegment mit einer für die Zehe vorgesehenen Kontaktfläche und einer gegenüberliegenden Auflagefläche ausgestaltet sein. In dem am Fuß befestigten Zustand der Fußorthese kann die Kontaktfläche des Zehensegments an der Zehe anliegen, insbesondere unmittelbar anliegen. Die Kontaktfläche kann hierbei in Form einer Wendefläche ausgestaltet sein, deren Orientierung, d.h. deren Flächennormale, sich entlang der Zehenlängsachse ändert und vorzugsweise auf die Zehenlängsachse zeigt. Auf diese Weise kann sich die Kontaktfläche und damit auch das Zehensegment entlang einer Schraubenlinie um die Zehenlängsachse erstrecken.

Um die erste Korrekturkraft in die Zehe einzuleiten oder auf diese auszuüben, umfasst das Zehensegment einen Zehenstützabschnitt, der in dem befestigten Zustand der Fußorthese anliegt an der lateralen Seite der Zehe, d.h. an der in lateraler Richtung zeigenden Seite. Mit anderen Worten kann der Zehenstützabschnitt dazu eingerichtet sein, die erste Korrekturkraft in die Zehe einzuleiten oder auf die Zehe auszuüben, wobei die erste Korrekturkraft in die mediale Richtung zeigt oder mit dieser zusammenfällt, insbesondere im Wesentlichen zusammenfällt. Der Zehenstützabschnitt kann durch einen Endabschnitt des Zehensegments gebildet sein, der insbesondere distal angeordnet sein kann.

Das Zehensegment kann ferner einen Zehenbasisabschnitt umfassen, der denjenigen Teil des Zehensegments bilden kann, der sich entlang der Zehenoberseite oder entlang der Zehenunterseite der zu behandelnden Zehe erstreckt. Mit anderen Worten kann der Zehenbasisabschnitt plantar und/oder dorsal angeordnet sein. Entsprechend kann in dem befestigten Zustand der Fußorthese der Zehenbasisabschnitt parallel oder im Wesentlichen parallel zu der natürlichen Auflagefläche des Fußes verlaufen. Gemäß einer Ausgestaltungsform kann in dem am Fuß befestigten Zustand die Zehe zumindest abschnittsweise auf dem Zehenbasisabschnitt aufliegen. Alternativ kann der Zehenbasisabschnitt zumindest abschnittsweise auf der Zehe aufliegen. Der Zehenbasisabschnitt ist vorzugsweise kraft- und/oder momentübertragend mit dem Zehenstützabschnitt verbunden, beispielsweise integral oder stoffschlüssig verbunden.

Der Zehenstützabschnitt kann in Form eines Schenkels ausgebildet sein oder einen Schenkel umfassen und insbesondere eine Biegefeder bilden. Insbesondere kann der Zehenstützabschnitt dünnwandig ausgebildet sein. Weiterhin kann der Zehenstützabschnitt senkrecht oder im Wesentlichen senkrecht zu dem Zehenbasisabschnitt angeordnet sein. Alternativ oder zusätzlich kann der Zehenstützabschnitt vertikal oder im Wesentlichen vertikal angeordnet sein.

Der Zehenstützabschnitt kann relativ zu der Zehe lateral angeordnet sein. Entsprechend kann der Zehenstützabschnitt unmittelbar auf die Zehe die erste Korrekturkraft ausüben, indem dieser auf die Zehe in medialer Richtung drückt. Alternativ kann der Zehenstützabschnitt ein Stützband umfassen oder bilden, insbesondere in Form eines Riemens, einer Schlaufe, eines Bandes, eines Gurtes, etc. Das Stützband kann an einer lateralen Seite der Zehe anliegen und auf diese die erste Korrekturkraft ausüben, wobei das Stützband an dessen Endabschnitten endsprechend mit einer Zugkraft beaufschlagt sein kann. Hierzu kann das Stützband an einem relativ zu der Zehe medial angeordneten Schenkel des Zehenstützabschnitts befestigt sein und/oder kraftübertragend mit dem Zehenbasisabschnitt verbunden sein. In dem befestigten Zustand der Fußorthese kann das Stützband zumindest teilweise um die Zehe angeordnet sein. Mit anderen Worten kann in dem befestigten Zustand die Zehe in dem Stützband derart angeordnet sein, dass das Stützband zumindest teilweise in Umfangrichtung an der Zehe anliegt. Das Stützband kann zugstarr oder elastisch ausgebildet sein, insbesondere entlang dessen Umfangsrichtung.

Das Zehensegment, insbesondere der Zehenbasisabschnitt und/oder der Zehenstützabschnitt, kann/können dünnwandig ausgebildet sein, um so zu einer kompakten Bauweise der Fußorthese beizutragen. Insbesondere kann/können das Zehensegment, insbesondere der Zehenbasisabschnitt und/oder der Zehenstützabschnitt, eine maximale Wanddicke von weniger als 3 mm oder weniger als 2 mm oder weniger als 1 mm aufweisen. Mit anderen Worten kann das Zehensegment, insbesondere der Zehenbasisabschnitt und/oder der Zehenstützabschnitt, aus wenigstens einem plattenförmigen Bauteil aufgebaut sein. Weiterhin kann/können das Zehensegment, insbesondere der Zehenbasisabschnitt und/oder der Zehenstützabschnitt, schalenförmig oder kalottenförmig ausgebildet sein und insbesondere in dessen Form an die Form der zu behandelnden Zehe angepasst sein. Auf diese Weise kann eine möglichst große Auflagefläche für die Zehe bereitgestellt sein, um das Tragen der Fußorthese für einen Patienten angenehmer zu gestalten. Weiterhin kann so eine kompakte Bauweise der Fußorthese sichergestellt werden.

Gemäß einer Weiterentwicklung kann das Zehensegment in Form eines Spannbügels ausgebildet sein. In dieser Ausgestaltung kann die erste Korrekturkraft in Form einer durch elastische Verformung des Zehensegments induzierten Spannkraft bereitgestellt sein.

Die Fußorthese kann derart ausgestaltet sein, dass in einem vom Fuß entkoppelten Zustand der Fußorthese, d.h. in einem Zustand, in dem die Fußorthese in keinem Eingriff mit dem zu behandelnden Fuß steht und entsprechend freiliegt, die Fußorthese, insbesondere das Zehensegment, in einer Ruheposition angeordnet ist, in der die Fußorthese, insbesondere das Zehensegment, nicht elastisch verformt ist. In dem am Fuß befestigten Zustand der Fußorthese kann die Fußorthese, insbesondere das Zehensegment, hingegen in einer Spannposition angeordnet sein, in der die Fußorthese, insbesondere das Zehensegment, elastisch verformt ist. Das Zehensegment, insbesondere der Zehenstützabschnitt, kann in der Spannposition gegenüber der Ruheposition in einer der ersten Korrekturkraft entgegengesetzten Richtung elastisch ausgelenkt sein. Beispielsweise kann der Zehenstützabschnitt, insbesondere ein Endabschnitt des Zehenstützabschnitts, in der Spannposition gegenüber der Ruheposition um wenigstens 0,2 cm, beispielsweise um wenigstens 0,3 cm oder wenigstens 0,5 cm oder wenigstens 1,0 cm, ausgelenkt oder translatorisch verschoben sein, insbesondere entlang der zu der ersten Korrekturkraft entgegengesetzten Richtung.

In der Spannposition kann das Zehensegment durch dessen elastische Verformung gespannt oder vorgespannt sein in Richtung der ersten Korrekturkraft. Die auf das Zehensegment, insbesondere auf den Zehenstützabschnitt, wirkende Spannkraft oder Biegekraft kann der ersten Korrekturkraft entsprechen. Alternativ kann die auf das Zehensegment, insbesondere auf den Zehenstützabschnitt, wirkende Spannkraft oder Biegekraft im Betrag im Wesentlichen der ersten Korrekturkraft entsprechen.

Die Fußorthese umfasst ferner das Ballensegment, das in dem am Fuß befestigten Zustand der Fußorthese im Bereich des Zehengrundgelenks angeordnet sein kann und insbesondere im Bereich des Zehengrundgelenks an dem Fuß anliegt. Mit anderen Worten ist das Ballensegment dafür vorgesehen, mit dem Fuß im Bereich des Zehengrundgelenks in Eingriff gebracht zu werden, um in dem am Fuß befestigten Zustand der Fußorthese eine kraftübertragende Kopplung zwischen dem Fuß im Bereich des Zehengrundgelenks und dem Ballensegment bereitzustellen. Entsprechend ist das Ballensegment in dem am Fuß befestigten Zustand an dem Fuß befestigt, insbesondere kraftschlüssig und/oder formschlüssig befestigt, und wird so an einer gewünschten Position an dem Fuß gehalten.

Genauer kann das Ballensegment derart ausgestaltet sein, dass in dem am Fuß befestigten Zustand das Ballensegment seitlich an dem Fuß im Bereich des Grundgelenkfußballens anliegt, insbesondere seitlich an dem Grundgelenkfußballen anliegt. Beispielsweise kann das Ballensegment in dem am Fuß befestigten Zustand an oder im Bereich von einer seitlichen, ballenförmigen Ausbuchtung des Fußes, insbesondere des ersten Mittelfußknochens, was auch als Pseudoexostose bezeichnet wird, anliegen. In einer Weiterentwicklung kann das Ballensegment derart ausgestaltet sein, dass in dem am Fuß befestigten Zustand das Ballensegment im Bereich des Grundgelenkfußballens anliegt, aber nicht an dem Grundgelenkfußballen oder einer Pseudoexostose selbst anliegt. Hierzu kann das Ballensegment mit einer Ausnehmung oder Durchgangsöffnung versehen sein, die in dem am Fuß befestigten Zustand wenigstens einen Teil des Grundgelenkfußballens, insbesondere die ballenförmige Ausbuchtung am Fuß, beispielsweise die Pseudoexostose, aufnimmt. Ein die Ausnehmung oder die Durchgangsöffnung begrenzender Teil des Ballensegments kann hierbei an dem Fuß in einem Bereich anliegen, der an dem Grundgelenkfußballen, insbesondere an der ballenförmigen Ausbuchtung am Fuß, beispielsweise an der Pseudoexostose, angrenzt. Mit anderen Worten kann die Fußorthese derart ausgestaltet sein, dass in dem am Fuß befestigten Zustand ein seitlich ausbuchtender Teil des Grundgelenkfußballens in der Ausnehmung oder der Durchgangsöffnung des Ballensegments aufgenommen ist. Beispielsweise kann das Ballensegment an dem Fuß umlaufend um den Grundgelenkfußballen, insbesondere um die ballenförmige Ausbuchtung am Fuß, beispielsweise um die Pseudoexostose, angeordnet sein. Die Ausnehmung oder Durchgangsöffnung an dem Ballensegment kann hierbei im Querschnitt einen Durchmesser, insbesondere minimalen oder maximalen Durchmesser, von wenigstens im Wesentlichen 1,5 cm oder von wenigstens im Wesentlichen 2,0 cm oder von wenigstens im Wesentlichen 2,5 cm aufweisen. Diese Ausgestaltung ermöglicht, dass die vorgeschlagene Fußorthese mittels des Ballensegments die zweite Korrekturkraft auf das Zehengrundgelenk ausüben kann, ohne hierfür unmittelbar auf schmerzempfindliche Bereiche des Fußes einwirken zu müssen, Kraft auszuüben oder zu berühren.

Im Zusammenhang mit der vorliegenden Offenbarung betrifft das Merkmal, dass die "zweite Korrekturkraft auf das Zehengrundgelenk ausgeübt wird", solche Korrekturkräfte, die auf das Zehengrundgelenk therapeutisch einwirken. Solche Korrekturkräfte können mittels der Fußorthese unmittelbar in das Zehengrundgelenk eingeleitet werden. Alternativ können solche Korrekturkräfte mittels der Fußorthese mittelbar auf das Zehengrundgelenk einwirken, beispielsweise indem das Ballensegment eine Korrekturkraft auf einen Mittelfußknochen, insbesondere den ersten Mittelfußknochen, ausübt. Die zweite Korrekturkraft hat insbesondere den Zweck, auf die Varusstellung eines Mittelfußknochens, im Speziellen auf die Varusstellung des ersten Mittelfußknochens, therapeutisch einzuwirken.

Zur Behandlung von Hallux valgus kann die Fußorthese derart bereitgestellt sein, dass das Ballensegment in dem am Fuß befestigten Zustand an dem Zehengrundgelenk an einer medialen Seite des Fußes anliegt. Die zweite Korrekturkraft kann entsprechend auf das Zehengrundgelenk in einer lateralen Richtung wirken.

Das Ballensegment kann dazu eingerichtet sein, in dem am Fuß befestigten Zustand Scherkräfte und/oder Biegekräfte, insbesondere in Richtung der zweiten Korrekturkraft, aufzunehmen und zu übertragen, insbesondere zwischen der Gelenkeinheit und dem zu behandelnden Zehengrundgelenk, um die zweite Korrekturkraft auf das Zehengrundgelenk auszuüben. Das Ballensegment kann insbesondere in Form eines Bügels bereitgestellt sein oder einen Teil eines Bügels bilden. Gemäß einer Ausgestaltungsform kann das Ballensegment den Grundgelenkfußballen zumindest abschnittsweise umgreifen. Hierzu kann sich das Ballensegment von einer lateralen Seite des Fußes im Bereich des Zehengrundgelenks in Richtung der Fußsohle erstrecken und insbesondere in seiner Form an die Form des Fußgewölbes angepasst sein. Alternativ kann sich das Ballensegment von der lateralen Seite des Fußes in Richtung des Fußrückens erstrecken. Beispielsweise kann sich das Ballensegment um den Grundgelenkfußballen über ein Bogenmaß von wenigstens 1/2 π rad um die Zehenlängsachse erstrecken.

Im Hinblick auf die geometrische Ausgestaltung ist das Ballensegment vorzugsweise plattenförmig oder schalenförmig ausgebildet. Beispielsweise kann das Ballensegment in seiner geometrischen Ausgestaltung riemen- oder bandförmig ausgebildet sein. Das Ballensegment ist vorzugsweise biegesteif. Entsprechend kann das Ballensegment mit einer für den Fuß vorgesehenen Kontaktfläche und einer gegenüberliegenden Auflagefläche ausgestaltet sein. In dem am Fuß befestigten Zustand der Fußorthese kann die Kontaktfläche des Ballensegments an dem Fuß anliegen, insbesondere unmittelbar anliegen. Die Kontaktfläche kann hierbei in Form einer Wendefläche ausgestaltet sein, deren Orientierung, d.h. deren Flächennormale sich entlang einer zu der Zehenlängsachse im Wesentlichen parallelen Längsachse ändert und vorzugsweise auf die Längsachse zeigt. Auf diese Weise kann sich die Kontaktfläche und damit auch das Ballensegment entlang einer Schraubenlinie um die Längsachse erstrecken.

Um die zweite Korrekturkraft in den Fuß einzuleiten oder auf diesen auszuüben, kann das Ballensegment einen Ballenstützabschnitt umfassen, der in dem befestigten Zustand der Fußorthese an dem Grundgelenkfußballen anliegen kann, insbesondere an einer medialen Seite des Fußes. Mit anderen Worten kann das Ballenstützelement dazu eingerichtet sein, die zweite Korrekturkraft auf das Zehengrundgelenk auszuüben, wobei die zweite Korrekturkraft in die laterale Richtung zeigt oder mit dieser zusammenfällt, insbesondere im Wesentlichen zusammenfällt. Der Ballenstützabschnitt kann durch einen Endabschnitt des Ballensegments gebildet sein.

Das Ballensegment kann ferner einen Ballenbasisabschnitt umfassen, der denjenigen Teil des Ballensegments bilden kann, der sich entlang der Fußsohle oder entlang des Fußrückens des zu behandelnden Fußes erstrecken kann. Mit anderen Worten kann der Ballenbasisabschnitt plantar und/oder dorsal angeordnet sein. Gemäß einer Ausführungsform kann in dem befestigten Zustand der Fußorthese der Ballenbasisabschnitt parallel oder im Wesentlichen parallel zu der natürlichen Auflagefläche des Fußes verlaufen. Gemäß einer Weiterentwicklung kann in dem am Fuß befestigten Zustand der Grundgelenkfußballen zumindest abschnittsweise auf dem Ballenbasisabschnitt aufliegen. Der Ballenbasisabschnitt ist vorzugsweise kraft- und/oder momentübertragend mit dem Ballenstützabschnitt verbunden, beispielsweise integral oder stoffschlüssig verbunden.

Der Ballenstützabschnitt kann in Form eines Schenkels ausgebildet sein oder einen Schenkel umfassen und insbesondere eine Biegefeder bilden. Insbesondere kann der Ballenstützabschnitt dünnwandig ausgebildet sein. Weiterhin kann der Ballenstützabschnitt vertikal angeordnet sein. Gemäß einer Ausführungsform kann der Ballenstützabschnitt senkrecht oder im Wesentlichen senkrecht zu dem Ballenbasisabschnitt angeordnet sein. Alternativ oder zusätzlich kann der Ballenstützabschnitt vertikal oder im Wesentlichen vertikal angeordnet sein.

Das Ballensegment, insbesondere der Ballenbasisabschnitt und/oder der Ballenstützabschnitt, kann/können dünnwandig ausgebildet sein, um so zu einer kompakten Bauweise der Fußorthese beizutragen. Insbesondere kann/können das Ballensegment, insbesondere der Ballenbasisabschnitt und/oder der Ballenstützabschnitt, eine maximale Wanddicke von weniger als 3 mm oder weniger als 2 mm oder von im Wesentlichen 1 mm aufweisen. Mit anderen Worten kann das Ballensegment, insbesondere der Ballenbasisabschnitt und/oder der Ballenstützabschnitt, aus wenigstens einem plattenförmigen Bauteil aufgebaut sein. Weiterhin kann/können das Ballensegment, insbesondere der Ballenbasisabschnitt und/oder der Ballenstützabschnitt, schalenförmig oder kalottenförmig ausgebildet sein und insbesondere in dessen Form an die Form des zu behandelnden Fu-βes angepasst sein.

Gemäß einer Weiterentwicklung kann das Ballensegment in Form eines Spannbügels ausgebildet sein. In dieser Ausgestaltung kann die zweite Korrekturkraft in Form einer durch elastische Verformung des Ballensegments induzierten Spannkraft bereitgestellt sein.

Die Fußorthese kann derart ausgestaltet sein, dass in dem vom Fuß entkoppelten Zustand der Fußorthese das Ballensegment in einer Ruheposition angeordnet ist, in der das Ballensegment nicht elastisch verformt ist. In dem am Fuß befestigten Zustand der Fußorthese kann das Ballensegment hingegen in einer Spannposition angeordnet sein, in der das Ballensegment elastisch verformt ist. Das Ballensegment, insbesondere der Ballenstützabschnitt, kann in seiner Spannposition gegenüber seiner Ruheposition in einer der zweiten Korrekturkraft entgegengesetzten Richtung elastisch ausgelenkt sein. Beispielsweise kann der Ballenstützabschnitt, insbesondere ein Endabschnitt des Ballenstützabschnitts, in der Spannposition gegenüber der Ruheposition um wenigstens 0,1 cm oder um wenigstens 0,2 cm oder um wenigstens 0,5 cm ausgelenkt oder translatorisch verschoben sein, insbesondere entlang der zu der zweiten Korrekturkraft entgegengesetzten Richtung.

In der Spannposition kann das Ballensegment durch dessen elastische Verformung gespannt oder vorgespannt sein in Richtung der zweiten Korrekturkraft. Die auf das Ballensegment, insbesondere auf den Ballenstützabschnitt, wirkende Spannkraft kann der zweiten Korrekturkraft entsprechen.

Die Fußorthese kann ferner ein Mittelfußsegment umfassen, das kraft- und momentübertragend mit dem Ballensegment verbunden sein kann. Das Mittelfußsegment kann dazu eingerichtet sein, die Fußorthese an dem Fuß in einem Mittelfußbereich zu befestigen. Das Mittelfußsegment kann in Form eines Bügels bereitgestellt sein, der in dem am Fuß befestigten Zustand der Fußorthese einen seitlichen Mittelfußbereich, insbesondere einen lateralen Mittelfußbereich, zumindest teilweise umgreift.

Das Mittelfußsegment kann dafür vorgesehen sein, in dem am Fuß befestigten Zustand eine Haltekraft auf den Mittelfuß auszuüben. Die Haltekraft kann in einer Richtung parallel zu der ersten Korrekturkraft wirken und zusammen mit der ersten Korrekturkraft eine Gegenkraft zu der zweiten Korrekturkraft bilden. Durch das Zusammenwirken dieser Kräfte kann die Fußorthese zuverlässig an dem zu behandelnden Fuß in einer für die therapeutische Behandlung vorgesehenen Position stabil gehalten werden. Durch diese Ausgestaltung kann die Fußorthese mit der Wirkungsweise einer Spannklemme oder -klammer an dem zu behandelnden Fuß befestigt werden.

In einer Weiterentwicklung kann die durch das Mittelfußsegment auszuübende Haltekraft eine therapeutische Wirkung auf den zu behandelnden Fuß haben, die insbesondere zur therapeutischen Wirkung der ersten und zweiten Korrekturkraft beiträgt und/oder eine davon zu unterscheidende, weitere therapeutische Wirkung bereitstellt. Beispielsweise kann die durch das Mittelfußsegment ausgeübte weitere Korrekturkraft ein Aufrichten des Fußgewölbes bewirken. Zur Unterstützung dieser Wirkung, kann die Fußorthese weiterhin ein unterhalb der Fußsohle, insbesondere des Fußgewölbes im Mittelfußbereich anzuordnendes Fußkissen, auch als Pelotte bezeichnet, umfassen. Ein derartiges Fußkissen kann lösbar mit dem Mittelfußsegment und/oder dem Ballensegment verbunden sein.

Das insbesondere als Bügel ausgebildete Mittelfußsegment kann dazu eingerichtet sein, in dem am Fuß befestigten Zustand Scherkräfte und/oder Biegekräfte, insbesondere in Richtung der Haltekraft, aufzunehmen und zu übertragen, insbesondere zwischen dem Ballensegment und dem zu behandelnden Mittelfuß, um die Haltekraft auszuüben.

Das als Bügel ausgebildete Mittelfußsegment ist derart ausgestaltet, dass in dem am Fuß befestigten Zustand das Mittelfußsegment den Mittelfußbereich in Umfangsrichtung teilweise umgreift. Mit anderen Worten erstreckt sich das Mittelfußsegment in dem am Fuß befestigten Zustand in Mittelfußumfangsrichtung entlang des Mittelfußes, vorzugsweise über ein Bogenmaß von wenigstens ½ π rad um eine Mittelfußlängsachse. Eine derartige Ausgestaltung ermöglicht, dass in dem am Fuß befestigten Zustand der Fußorthese das Ballensegment, insbesondere der Ballenstützabschnitt, gegenüberliegend zu dem Mittelfußsegment, insbesondere einem Endabschnitt des Mittelfußsegments, angeordnet ist, um einen stabilen Halt der Fußorthese an dem zu behandelnden Fuß zu gewährleisten.

Im Hinblick auf die geometrische Ausgestaltung ist das als Bügel ausgebildete Mittelfußsegment vorzugsweise plattenförmig ausgebildet. Beispielsweise kann das Mittelfußsegment in seiner geometrischen Ausgestaltung riemen- oder bandförmig ausgebildet sein und insbesondere biegesteif sein. Entsprechend kann das Mittelfußsegment mit einer für den Mittelfuß vorgesehenen Kontaktfläche und einer gegenüberliegenden Auflagefläche ausgestaltet sein. In dem am Fuß befestigten Zustand kann der Mittelfuß an der Kontaktfläche des Mittelfußsegments anliegen, insbesondere unmittelbar anliegen.

Um die Haltekraft in den Mittelfuß einzuleiten oder auf diesen auszuüben, kann das Mittelfußsegment einen Mittelfußstützabschnitt umfassen, der in dem befestigten Zustand der Fußorthese an dem Mittelfuß anliegt, insbesondere an einer lateralen Seite. Mit anderen Worten kann das Mittelfußsegment dazu eingerichtet sein, die Haltekraft in den Mittelfuß einzuleiten oder darauf auszuüben, wobei die Haltekraft in die mediale Richtung zeigt oder mit dieser zusammenfällt, insbesondere im Wesentlichen zusammenfällt. Der Mittelfußstützabschnitt kann durch einen Endabschnitt des Mittelfußsegments gebildet sein.

Das Mittelfußsegment kann ferner einen Mittelfußbasisabschnitt umfassen, der sich entlang der Fußsohle oder des Fußrückens des zu behandelnden Fußes erstrecken kann. Mit anderen Worten kann der Mittelfußbasisabschnitt plantar und/oder dorsal angeordnet sein. Gemäß einer Ausführungsform kann der Mittelfußbasisabschnitt in seiner geometrischen Ausgestaltung an die Form des Fußgewölbes des zu behandelnden Fußes angepasst sein. Der Mittelfußbasisabschnitt ist vorzugsweise kraft- und/oder momentübertragend mit dem Mittelfußstützabschnitt verbunden, beispielsweise integral oder stoffschlüssig verbunden.

Der Mittelfußstützabschnitt kann in Form eines Schenkels ausgebildet sein oder einen Schenkel umfassen und insbesondere eine Biegefeder bilden. Insbesondere kann der Mittelfußstützabschnitt dünnwandig ausgebildet sein. Weiterhin kann der Mittelfußstützabschnitt im Wesentlichen vertikalangeordnet sein.

Der Mittelfußstützabschnitt kann relativ zu dem Mittelfuß lateral angeordnet sein. Entsprechend kann der Mittelfußstützabschnitt unmittelbar auf den Mittelfuß die Haltekraft ausüben, indem dieser auf den Mittelfuß in medialer Richtung drückt. Alternativ kann der Mittelfußstützabschnitt ein Stützband umfassen oder bilden, insbesondere in Form eines Riemens, einer Schlaufe, eines Bandes, eines Gurtes etc. Das Stützband kann an einer lateralen Seite des Mittelfußes anliegen und auf diesen die Haltekraft ausüben, wobei das Stützband an dessen Endabschnitten endsprechend mit einer Zugkraft beaufschlagt sein kann und mit dem Mittelfußsegment über dessen Endabschnitt der verbunden sein kann. Hierzu kann das Stützband an einem relativ zu dem Mittelfuß medial angeordneten Schenkel des Mittelfußstützabschnitts befestigt sein und/oder kraftübertragend mit dem Mittelfußbasisabschnitt verbunden sein. In dem befestigten Zustand der Fußorthese kann das Stützband zumindest teilweise in Umfangsrichtung um den Mittelfußbereich angeordnet sein. Mit anderen Worten kann in dem befestigten Zustand der Mittelfuß in dem Stützband derart angeordnet sein, dass das Stützband zumindest teilweise in Umfangsrichtung an dem Mittelfuß anliegt. Das Stützband kann zugstarr oder elastisch ausgebildet sein, insbesondere entlang dessen Umfangsrichtung.

Das Mittelfußsegment, insbesondere der Mittelfußbasisabschnitt und/oder der Mittelfußstützabschnitt, kann/können dünnwandig ausgebildet sein, um so zu einer kompakten Bauweise der Fußorthese beizutragen. Insbesondere kann/können das Mittelfußsegment, insbesondere der Mittelfußbasisabschnitt und/oder der Mittelfußstützabschnitt, eine maximale Wanddicke von weniger als 3 mm oder weniger als 2 mm oder im Wesentlichen 1 mm aufweisen. Mit anderen Worten kann das Mittelfußsegment, insbesondere der Mittelfußbasisabschnitt und/oder der Mittelfußstützabschnitt, aus wenigstens einem plattenförmigen oder schalenförmigen Bauteil aufgebaut sein. Weiterhin kann/können das Mittelfußsegment, insbesondere der Mittelfußbasisabschnitt und/oder der Mittelfußstützabschnitt, schalenförmig oder kalottenförmig ausgebildet sein und insbesondere in dessen Form an die Form des zu behandelnden Mittelfußes angepasst sein.

Gemäß einer Weiterentwicklung kann das Mittelfußsegment in Form eines Spannbügels ausgebildet sein. In dieser Ausgestaltung kann die Haltekraft in Form einer durch elastische Verformung des Mittelfußsegments induzierten Spannkraft oder Biegekraft bereitgestellt sein.

Die Fußorthese kann derart ausgestaltet sein, dass in dem vom Fuß entkoppelten Zustand der Fußorthese das Mittelfußsegment in einer Ruheposition angeordnet ist, in der das Mittelfußsegment nicht elastisch verformt ist. In dem am Fuß befestigten Zustand der Fußorthese kann das Mittelfußsegment hingegen in einer Spannposition angeordnet sein, in der das Mittelfußsegment elastisch verformt ist. Das Mittelfußsegment, insbesondere der Mittelfußstützabschnitt, kann in seiner Spannposition gegenüber seiner Ruheposition in einer der Haltekraft entgegengesetzten Richtung elastisch ausgelenkt sein. Beispielsweise kann der Mittelfußstützabschnitt, insbesondere ein Endabschnitt des Mittelfußstützabschnitts, in der Spannposition gegenüber der Ruheposition um wenigstens 0,3 cm, beispielsweise um wenigstens 0,5 cm oder um wenigstens 1,0 cm, ausgelenkt oder translatorisch verschoben sein, insbesondere entlang der zu der Haltekraft entgegengesetzten Richtung.

In der Spannposition kann das Mittelfußsegment durch dessen elastische Verformung gespannt oder vorgespannt sein in Richtung der Haltekraft. Die auf das Mittelfußsegment, insbesondere auf den Mittelfußstützabschnitt, wirkende Spannkraft kann der Haltekraft entsprechen. Alternativ kann die auf das Mittelfußsegment, insbesondere auf den Mittelfußstützabschnitt, wirkende Spannkraft im Betrag im Wesentlichen der Haltekraft entsprechen.

Das Zehensegment und/oder das Ballensegment und/oder das Mittelfußsegment kann/können aus einem Kunststoff hergestellt sein, insbesondere aus einem Thermoplast oder einem thermoplastischen Elastomer, etc. Das Zehensegment und/oder das Ballensegment und/oder das Mittelfußsegment kann/können durch ein additives Fertigungsverfahren oder ein Spritzgussverfahren hergestellt sein. Auch können die einzelnen Segmente unterschiedliche Materialien, insbesondere Kunststoffe umfassen, die vorzugsweise unterschiedliche Werkstoffeigenschaften aufweisen. Die Verwendung eines additiven Fertigungsverfahren odere eines Spritzgussverfahrens ermöglicht hierbei, dass die einzelnen Segmente integral ausgebildet sein können und trotzdem aus unterschiedlichen Materialien bestehen können und unterschiedliche Materialeigenschaften aufweisen können. Mit anderen Worten können die aus unterschiedlichen Materialien bestehenden Teile der einzelnen Segmente integral oder stoffschlüssig miteinander verbunden sein.

Gemäß einer Weiterentwicklung können unterschiedliche Teile der einzelnen Segmente unterschiedlich steif ausgestaltet sein. Beispielsweise kann vorgesehen sein, dass der jeweilige Stützabschnitt der unterschiedlichen Segmente gegenüber dessen Basisabschnitt mit einer geringeren Steifigkeit versehen sein kann. Mit anderen Worten können/kann das Zehensegment und/oder das Ballensegment und/oder das Mittefußsegment einen Basisabschnitt und einen in dem am Fuß befestigten Zustand die erste Korrekturkraft oder die zweite Korrekturkraft oder eine Haltekraft in den Fuß einleitenden Stützabschnitt aufweisen, wobei der Stützabschnitt gegenüber dem Basisabschnitt eine geringere Steifigkeit aufweist, insbesondere gegenüber Scher- und/oder Biegekräften in Richtung der ersten oder zweiten Korrekturkraft. Genauer kann/können der Zehenstützabschnitt eine geringere Steifigkeit gegenüber dem Zehenbasisabschnitt und/oder der Ballenstützabschnitt eine geringere Steifigkeit gegenüber dem Ballenbasisabschnitt und/oder der Mittelfußstützabschnitt eine geringere Steifigkeit gegenüber dem Mittelfußbasisabschnitt aufweisen. Hierzu kann die geometrische Ausgestaltung der einzelnen Segmente gezielt angepasst werden, indem beispielsweise der Stützabschnitt eine geringere Materialdicke aufweist als der dazu korrespondierende Basisabschnitt. Alternativ oder zusätzlich können gezielt unterschiedliche Werkstoffe für die einzelnen Segmente verwendet werden. Beispielsweise kann der Stützabschnitt aus einem Material hergestellt sein, das ein geringeres Elastizitätsmodul und/oder eine geringere Festigkeit und/oder geringere Härte aufweist als das Material, aus welchem der dazu korrespondierende Basisabschnitt hergestellt ist.

In einer Weiterentwicklung können der Zehenstützabschnitt und der Zehenbasisabschnitt integral ausgebildet sein. Alternativ oder zusätzlich können der Ballenstützabschnitt und der Ballenbasisabschnitt integral ausgebildet sein. Alternativ oder zusätzlich können der Mittelfußstützabschnitt und der Mittelfußbasisabschnitt integral ausgebildet sein.

Alternativ oder zusätzlich kann die Fußorthese ein an der Ferse oder hinteren Teil des zu behandelnden Fußes befestigbares Fersensegment umfassen, das in dem am Fuß befestigten Zustand eine Haltekraft auf den hinteren Teil des Fußes ausübt. Das Fersensegment kann alternativ oder zusätzlich zu dem Mittelfußsegment bereitgestellt sein und mit entsprechenden Eigenschaften ausgestaltet sein. Mit anderen Worten können die vorliegend im Zusammenhang mit dem Mittelfußabschnitt beschriebenen Merkmale auch als für das Fersensegment offenbart angesehen werden.

Wie vorangehend beschrieben, sind das Zehensegment und das Ballensegment mittels einer Gelenkeinheit relativ zueinander verschwenkbar gekoppelt. Indem die vorgeschlagene Fußorthese mit dem das Zehensegment und das Ballensegment miteinander schwenkbar verbindenden Gelenkeinheit ausgestaltet ist, kann eine mittels der vorgeschlagenen Fußorthese geschiente Zehe entlang der Flexion-Extension-Bewegungsrichtung bewegt werden. Mit anderen Worten ist die Gelenkeinheit derart eingerichtet sein, dass in dem am Fuß befestigten Zustand die zu behandelnde Zehe relativ zu dem Großzehengrundgelenk in Flexion-und Extensionsrichtung bewegbar ist. Entsprechend ist die Gelenkeinheit derart eingerichtet, dass das Zehensegment und das Ballensegment relativ zueinander um eine Schwenkachse verschwenkbar sind, wobei die Schwenkachse im Wesentlichen parallel, insbesondere parallel, zu einer Gelenkachse des Zehengrundgelenks in Flexions- und Extensionsrichtung ist. Zusätzlich kann die Schwenkachse parallel oder im Wesentlichen parallel zu der ersten und zweiten Korrekturkraft sein. Die Schwenkachse kann weiterhin mit der Gelenkachse fluchten oder im Wesentlichen fluchten.

Weiterhin kann die Gelenkeinheit dazu eingerichtet sein, Scher- und/oder Biegekräfte zwischen dem Zehensegment und dem Ballensegment zu übertragen, um die erste und/oder zweite Korrekturkraft auf den Fuß auszuüben. Mit anderen Worten kann die Gelenkeinheit der Fußorthese dazu eingerichtet sein, in dem befestigten Zustand parallel zu den Korrekturkräften wirkende Kräfte, insbesondere Scherkräfte oder Schubkräfte, zu übertragen, um die erste und/oder zweite Korrekturkraft zu generieren. Mit anderen Worten ist die gesamte Fußorthese dazu geeignet, innere Kräfte beziehungsweise Spannungen entlang der Fußorthesen-Längsachse in Richtung der Korrekturkräfte zu übertragen. Entsprechend kann die Gelenkeinheit derart ausgebildet sein, dass in dem am Fuß befestigten Zustand der Fußorthese eine relative Schwenkbewegung zwischen dem Zehensegment und dem Ballensegment relativ zueinander um eine zu der Schwenkachse orthogonal angeordnete Achse gesperrt ist.

Auf diese Weise räumt die vorgeschlagene Fußorthese einer geschienten Zehe eine ausreichende Bewegungsfreiheit ein, sodass die Fußorthese den Fuß in seiner natürlichen Gehbewegung unterstützt und gleichzeitig therapeutisch auf diesen einwirkt. Dies ermöglicht den Einsatz der vorgeschlagenen Fußorthese im Alltag des Patienten, was dessen Bereitschaft zum Tragen der Fußorthese und daher die Akzeptanz und damit einhergehend den Erfolg der therapeutischen Behandlung erhöht.

In einer Weiterentwicklung kann die Gelenkeinheit durch miteinander strukturell im Eingriff stehende Bereiche, insbesondere Endabschnitte, des Zehensegments und des Ballensegments gebildet sein. Genauer kann die Gelenkeinheit durch im Eingriff stehende Bereiche, insbesondere Endabschnitte, des Zehenbasisabschnitts und des Ballenbasisabschnitts ausgebildet sein. Die Gelenkeinheit kann wenigstens teilweise durch die im Eingriff stehende Bereiche des Zehensegments und des Ballensegments gebildet sein oder ausschließlich durch die im Eingriff stehende Bereiche des Zehensegments und des Ballensegments gebildet sein. Der die Gelenkeinheit bildende Teil des Zehensegments und/oder des Ballensegments kann integraler Bestandteil des Zehensegments, insbesondere des Zehenbasisabschnitts, und/oder des Ballensegments, insbesondere des Ballenbasisabschnitts sein. Auf diese Weise kann ein einfacher Aufbau der Fußorthese durch die Verwendung einer geringen Anzahl von Komponenten sichergestellt werden.

Gemäß einer Ausführungsform kann die Gelenkeinheit in dem am Fuß befestigten Zustand seitlich am Fuß angeordnet sein. Insbesondere kann die Gelenkeinheit an einer medialen Seite des Fußes in dem befestigten Zustand angeordnet sein. Hierbei kann die mit der Schwenkachse zusammenfallende Gelenkachse der Gelenkeinheit mit der Gelenkachse des Zehengrundgelenks zusammenfallen oder im Wesentlichen zusammenfallen. Gemäß dieser Ausführungsform kann der die Gelenkeinheit bildende Teil des Zehensegments und/oder der dazu korrespondierende, die Gelenkeinheit bildende weitere Teil des Ballensegments im Bereich des zu behandelnden Zehengrundgelenks angeordnet sein und die zweite Korrekturkraft auf das Zehengrundgelenk ausüben.

Alternativ oder zusätzlich kann die Gelenkeinheit in dem am Fuß befestigten Zustand im Bereich der Fußsohle und/oder des Fußrückens angeordnet sein. Entsprechend kann die Gelenkeinheit sich entlang der Fußsohle und/oder entlang des Fußrückens erstrecken, insbesondere entlang einer zu der ersten und zweiten Korrekturkraft parallelen oder im Wesentlichen parallelen Richtung.

In einer Weiterentwicklung kann die Gelenkeinheit wenigstens ein Drehgelenk und/oder ein Biegegelenk umfassen. Im Zusammenhang mit der vorliegenden Offenbarung wird als Drehgelenk ein Gelenk verstanden, bei dem zwei relativ zueinander verschwenkbar und in Eingriff gebrachte Teile des Gelenks relativ zueinander verschwenkbar oder rotierbar gelagert sind. Demgegenüber beschreibt ein Biegegelenk ein Gelenk, bei dem zwei im Eingriff miteinander stehende Komponenten des Gelenks durch elastische oder plastische Verformung relativ zueinander verschwenkbar sind.

Das Drehgelenk kann in Form eines Ringgelenks bereitgestellt sein, bei dem eine Kontaktfläche oder eine Gleitfläche zwischen relativ zueinander verschwenkbaren Teilen des Drehgelenks ringförmig um die Gelenk- oder Schwenkachse des Drehgelenks angeordnet ist. Auf diese Weise kann die Gelenkeinheit besonders robust gegenüber Biegekräften und Biegemomenten sein, sodass die Gelenkeinheit hohe Kräfte und Momente übertragen und in den zu behandelnden Fuß einleiten kann, bei einer gleichzeitig kompakten Bauweise der Fußorthese.

Gemäß einer Weiterentwicklung kann das Drehgelenk, insbesondere nach Art eines Ringgelenks, in Form eines Hohlzapfendrehgelenks bereitgestellt sein. Im Zusammenhang mit der vorliegenden Offenbarung betrifft der Begriff "Hohlzapfendrehgelenk", das auch als nabenloses Drehgelenk bezeichnet werden kann, ein Gelenk, das entlang seiner Schwenkachse zumindest abschnittsweise hohl ausgebildet ist, um eine Ausnehmung auszubilden. Mit anderen Worten sind die das Drehgelenk bildenden Komponenten entlang der Schwenkachse hohl ausgebildet, sodass das Drehgelenk um und entlang dessen Schwenkachse mit der Ausnehmung oder einer Durchgangsöffnung versehen ist.

Das Drehgelenk kann relativ zueinander um die Schwenkachse verschwenkbare Komponenten aufweisen, die relativ zueinander in dem Drehgelenk geführt sind. Der für die Führung der Komponenten vorgesehene Bereich, der durch Lagerpunkte und/oder Lagerflächen, insbesondere durch Kontaktflächen und/oder Gleitflächen, gebildet sein kann, ist vorzugsweise umlaufend um die Schwenkachse und beabstandet zu dieser angeordnet.

Der für die Führung der Komponenten des Drehgelenks vorgesehene Bereich kann um einen Führungsradius beabstandet zu der Schwenkachse angeordnet sein. Der Führungsradius kann insbesondere einen mittleren Radius des für die Führung der Komponenten des Drehgelenks vorgesehenen Bereichs um die Schwenkachse angeben. Der Führungsradius kann im Wesentlichen im Bereich eines Außenradius des Drehgelenks liegen, der eine Erstreckung des Drehgelenks in radialer Richtung beschreibt. Der Führungsradius kann wenigstens 70% des Außenradius des Drehgelenks betragen. Im Speziellen kann der Führungsradius wenigstens 80% oder wenigstens 90% des Außenradius betragen.

Das Drehgelenk kann derart ausgebildet sein, dass in dem befestigten Zustand der Fußorthese das Drehgelenk derart an dem Fuß und dem Zehengrundgelenk angeordnet ist, dass das Drehgelenk umlaufend um den seitlich ausbuchtenden Teil des Zehengrundgelenks, beispielsweise um die Pseudoexostose, angeordnet sein kann, wobei der seitlich ausbuchtende Teil des Zehengrundgelenks zumindest teilweise in der Ausnehmung des Drehgelenks angeordnet ist.

Die Ausnehmung kann als zumindest einseitig geöffnete Ausnehmung bereitgestellt sein. In dem am Fuß befestigten Zustand kann die Ausnehmung in Richtung des Fußes hin geöffnet sein. Alternativ kann die Ausnehmung in Form einer Durchgangsöffnung, insbesondere entlang der Schwenkachse ausgebildet sein. Mit anderen Worten kann sich die Ausnehmung entlang der gesamten Breite oder Dicke des Drehgelenks erstrecken. Vorliegend betreffen die Begriffe "Breite" oder "Dicke" des Drehgelenks eine Erstreckung des Drehgelenks entlang der Schwenkachse. Das Drehgelenk kann eine Breite, insbesondere maximale Breite, von höchstens 1,0 cm oder von höchstens 0,6 cm aufweisen.

Die Fußorthese kann derart ausgestaltet sein, dass in dem befestigten Zustand der seitlich ausbuchtende Teil des Zehengrundgelenks sich entlang wenigstens 50% oder wenigstens 70% oder wenigstens 80% der maximalen Breite des Drehgelenks erstreckt. Weiterhin kann die Fußorthese derart ausgestaltet sein, dass in dem befestigten Zustand der seitlich ausbuchtende Teil des Zehengrundgelenks durch die Ausnehmung, insbesondere die Durchgangsöffnung, entlang der Schwenkachse hindurchragt oder im Wesentlichen hindurchragt. Auf diese Weise kann die Fußorthese besonders eng am Fuß geführt werden.

Das Drehgelenk kann eine die Ausnehmung begrenzende und radial um die Schwenkachse angeordnete Seitenwand umfassen. Die Seitenwand kann einen minimalen Krümmungsradius von 1 mm oder 2 mm oder 5 mm aufweisen. Mit anderen Worten kann das Drehgelenk derart ausgebildet sein, dass die Seitenwand an keiner Stelle einen Krümmungsradius aufweist, der unterhalb des minimalen Krümmungsradius liegt. Der Kehrwert des Krümmungsradius entspricht hierbei der Krümmung der Seitenwand, insbesondere deren der Ausnehmung zugewandten Innenfläche. Auf diese Weise kann sichergestellt werden, dass der an dem seitlich ausbuchtenden Teil des Zehengrundgelenks anliegende Bereich des Drehgelenks nicht mit spitzen Kanten versehen ist, um so Druckspitzen am Fuß des Patienten beim Tragen der Fußorthese zu verhindern.

Die Ausnehmung kann einen minimalen Durchmesser, insbesondere entlang einer Richtung quer zur Schwenkachse oder um die Schwenkachse, von wenigstens 1,5 cm oder wenigstens 2,0 cm oder wenigstens 2,5 cm betragen. Beispielsweise kann die Ausnehmung im Querschnitt entlang der Schwenkachse eine kreisförmige oder elliptische Form aufweisen, deren minimaler Durchmesser wenigstens 1,5 cm oder wenigstens 2,0 cm oder wenigstens 2,5 cm betragen kann. Beispielsweise kann der Durchmesser 3,0 cm oder im Wesentlichen 3,0 cm betragen.

Die Form der Ausnehmung, insbesondere deren Querschnittsform und Durchmesser, kann an den zu behandelnden Fuß, insbesondere an die Form des seitlich ausbuchtenden Teils des Zehengrundgelenks, angepasst sein. Dies kann aufgrund orthopädischer beziehungsweise physiologischer Klassifikation anwendergruppenspezifisch erfolgen. Beispielsweise können auf diese Weise Fußorthesen für Anwendergruppen mit unterschiedlich großen Füßen und/oder unterschiedlich gro-βen seitlich ausbuchtenden Teilen des Zehengrundgelenks beziehungsweise Pseudoexostosen bereitgestellt werden.

Die Schwenkachse des Drehgelenks kann mit der Grundgelenkachse des Zehengrundgelenks, insbesondere mit der Flexions-Extensions-Bewegungsachse, fluchten, d.h. mit dieser zusammenfallen, oder im Wesentlichen fluchten. Alternativ oder zusätzlich kann die Schwenkachse des Drehgelenks parallel oder im Wesentlichen parallel zu der ersten und/oder zweiten Korrekturkraft angeordnet sein.

Das Drehgelenk kann derart ausgebildet sein, dass in dem am Fuß befestigten Zustand der Fußorthese eine relative Schwenkbewegung zwischen dem Zehensegment und dem Ballensegment um eine zu der Schwenkachse schräg oder orthogonal angeordnete Achse gesperrt ist. Das Drehgelenk kann derart ausgebildet sein, dass eine relative Schwenkbewegung nur um die Schwenkachse zugelassen ist. Mit anderen Worten kann das Drehgelenk strukturell derart ausgebildet sein, dass Schwenkbewegungen um die Schwenkachse erfolgen können, aber Schwenkbewegungen um eine Achse schräg oder quer zu der Schwenkachse gesperrt sind. Auf diese Weise kann eine einfache und kompakte Bauweise des Drehgelenks bereitgestellt sein.

Die Gelenkeinheit, insbesondere das Drehgelenk, kann ein mit dem Zehensegment gekoppeltes, insbesondere integral oder stoffschlüssig verbundenes, erstes Gelenkelement und ein dazu komplementär ausgebildetes und im Eingriff stehendes zweites Gelenkelement umfassen, das mit dem Ballensegment gekoppelt ist, insbesondere integral oder stoffschlüssig verbunden ist. Das erste Gelenkelement und das zweite Gelenkelement können entlang der Schwenkachse, insbesondere in einer ersten und einer dazu entgegengesetzten zweiten Richtung entlang der Schwenkachse, und/oder quer zu der Schwenkachse des Drehgelenks formschlüssig im Eingriff stehen.

Das Drehgelenk kann derart ausgestaltet sein, dass in dem befestigten Zustand das zweite Gelenkelement zwischen dem Fuß und dem ersten Gelenkelement angeordnet ist. Auf diese Weise kann verhindert werden, dass bei einer Beugebewegung der geschienten Zehe, der an dem Fuß anliegende Teil des Drehgelenks relativ zu dem seitlich ausbuchtenden Teil verschwenkt wird. Dies kann den Tragekomfort der Fußorthese erhöhen.

Das zweite Gelenkelement kann einen Gelenkzapfen des Drehgelenks bilden, der das einen Gelenkring bildende erste Gelenkelement um die Schwenkachse führt. Alternativ kann das erste Gelenkelement den Gelenkzapfen bilden und das zweite Gelenkelement den Gelenkring. Der Gelenkzapfen ist vorzugsweise als Hohlzapfen ausgebildet, wobei der hohle Teil des Hohlzapfens die Ausnehmung bildet. Der Gelenkring und der Gelenkzapfen können derart ausgebildet sein und im Eingriff stehen, dass diese entlang der Schwenkachse, insbesondere in der ersten und der dazu entgegengesetzten zweiten Richtung entlang der Schwenkachse, formschlüssig im Eingriff stehen.

Der Gelenkzapfen kann in seiner geometrischen Ausgestaltung an die Form des Gelenkrings angepasst sein. Der Gelenkring kann eine erste Führungsfläche aufweisen, die korrespondierend zu einer zweiten Führungsfläche des Gelenkzapfens ausgestaltet sein kann. Die erste und zweite Führungsfläche, die insbesondere Gleit- und Lagerflächen bilden, können im Eingriff stehen, insbesondere im Wesentlichen spielfrei oder mit einem vorgegebenen Spiel. Bei einer Schwenkbewegung können die erste und zweite Führungsfläche relativ zueinander bewegt werden.

Die erste Führungsfläche des Gelenkrings kann eine radial nach innen gerichtete, d.h. der Schwenkachse zugewandte, Fläche sein oder umfassen. Die erste Führungsfläche kann sich entsprechend in Umfangsrichtung um die Schwenkachse erstrecken und ringförmig um die Schwenkachse angeordnet sein.

Die zweite Führungsfläche des Gelenkzapfens kann eine radial nach außen gerichtete Fläche sein oder umfassen und insbesondere eine Mantelfläche des Gelenkzapfens bilden. Die zweite Führungsfläche kann sich entsprechend in Umfangsrichtung um die Schwenkachse erstrecken und ringförmig um die Schwenkachse angeordnet sein.

Der Gelenkzapfen kann ferner an einem distalen und/oder proximalen Ende einen umlaufenden radialen Absatz umfassen, welcher, insbesondere im Sinne eines Schnapphakens, in Richtung der

Drehachse eine formschlüssige Verbindung bzw. hinterschnittige Sicherung zwischen dem Gelenkring und Gelenkzapfen bereitstellt. Um an der medialen Außenseite der Fußorthese eine im Wesentlichen ebene Außenkontur bzw. Außenfläche bereitzustellen, kann der radiale Absatz in einer korrespondierend ausgebildeten Aufnahme oder Ausnehmung am Gelenkring aufgenommen sein. Alternativ oder zusätzlich kann ein separater Sicherungsring vorgesehen sein, welcher in eine entsprechende Nut am Gelenkring oder Gelenkzapfen eingesetzt werden kann. Der radiale Absatz kann sich entlang der Schwenkachse derart erstrecken, dass dieser in Axialrichtung des Drehgelenks überlappend zu dem Gelenkring angeordnet ist und diesen insbesondere umgreift. Durch diese Ausgestaltung kann verhindert werden, dass der zu behandelnde Fuß mit der Aufnahmenut oder dem Verbindungsring in Kontakt tritt, um den Tragekomfort für einen Patienten zu erhöhen.

Die erste und zweite Führungsfläche können wenigstens eine, beispielsweise zwei gegenüberliegenden axial begrenzende Seitenflächen umfassen, um die formschlüssige Verbindung entlang der Schwenkachse bereitzustellen. Auf diese Weise können Kräfte, insbesondere Biegekräfte in Richtung der Schwenkachse des Drehgelenks zwischen den Komponenten übertragen werden.

Gemäß einer Ausgestaltung kann der Gelenkzapfen, insbesondere das zweite Gelenkelement, oder der Gelenkring mit einer um die Schwenkachse umlaufend angeordneten Aufnahmenut versehen sein, die sich in radialer Richtung erstreckt, d.h. zu der Schwenkachse hin erstreckt, und in axialer Richtung entlang der Schwenkachse begrenzt ist, d.h. seitlich begrenzt ist. Die Innenflächen der Aufnahmenut bilden hierbei eine Kontakt- und Gleitfläche, d.h. die erste oder zweite Führungsfläche. Mit anderen Worten kann die Aufnahmenut im Längsschnitt entlang der Schwenkachse eine im Wesentlichen U-förmige Kontakt- oder Gleitfläche ausbilden. Die dazu korrespondierende Führungsfläche kann ein zu der Aufnahmenut korrespondierend ausgebildeter Verbindungsring sein, der in der Aufnahmenut geführt ist und relativ zu der Aufnahmenut in Umfangsrichtung um die Schwenkachse verdrehbar ist. In dem im Eingriff stehenden Zustand der Aufnahmenut und des Verbindungsrings ist der Verbindungsring derart in der Aufnahmenut angeordnet, dass das erste Gelenkelement und das zweite Gelenkelement formschlüssig in Axialrichtung der Schwenkachse miteinander verbunden sind.

Mit anderen Worten kann der Gelenkzapfen mit einer Aufnahmenut versehen sein, in der ein dazu korrespondierender Verbindungsring des Gelenkrings geführt ist. Alternativ kann der Gelenkring mit der Aufnahmenut versehen sein, in der der dazu korrespondierender Verbindungsring des Gelenkzapfens geführt ist. Die Gelenkeinheit kann derart ausgestaltet sein, dass in dem am Fuß befestigten Zustand der Fußorthese, das zweite Gelenkelement zwischen dem Fuß und dem ersten Gelenkelement angeordnet ist, wobei ein Verbindungssteg des zweiten Gelenkelements, der die Aufnahmenut ausbildet, die Ausnehmung oder Durchgangsöffnung des Drehgelenks umlaufend, insbesondere radial, begrenzt. Der Verbindungssteg kann sich entlang der Schwenkachse derart erstrecken, dass der Verbindungssteg in Axialrichtung der Gelenkeinheit überlappend zu dem ersten Gelenkelement, insbesondere dem Verbindungsring, angeordnet ist und insbesondere umgreift. Durch diese Ausgestaltung kann verhindert werden, dass der zu behandelnde Fuß mit der Aufnahmenut oder dem Verbindungsring in Kontakt tritt, um den Tragekomfort für einen Patienten zu erhöhen.

Gemäß einer Weiterentwicklung kann die die Bewegungsfreiheit sicherstellende Gelenkeinheit entlang des Fußrückens und/oder entlang der Fußsohle in dem am Fuß befestigten Zustand angeordnet sein. Mit anderen Worten kann die Gelenkeinheit plantar, d.h. fußsohlenseitig oder im Bereich der Fußsohle, und/oder dorsal, d.h. fußrückenseitig oder im Bereich des Fußrückens, am Fuß angeordnet sein. Dies ermöglicht eine konstruktiv einfache und platzsparende Ausgestaltung der Fußorthese.

Genauer kann sich die dorsal und/oder plantar anzuordnende Gelenkeinheit entlang einer Fußbreitenrichtung oder parallel zu der ersten und zweiten Korrekturkraft erstrecken, insbesondere entlang der gesamten Breite des Zehenbasissegments und/oder Ballenbasissegments. Durch eine solche Ausgestaltung kann/können niedrigaufbauende, also flache, Formen des Zehenbasissegments und/oder Ballenbasissegment erzielt werden. Entsprechend können das Zehenbasissegment und/oder Ballenbasissegment hohe Flächenträgheitsmomente gegenüber in Richtung der Korrekturkräfte wirkenden Beanspruchungen bei gleichzeitig geringer Querschnittsfläche aufweisen, was in besonderem Maße zu einer kompakten Bauweise der Fußorthese beitragen kann.

Die dorsale und/oder plantar anzuordnende Gelenkeinheit kann in Form eines Biegegelenks, insbesondere als Eingelenkscharnier, beispielsweise in Form eines Filmscharniers, ausgebildet sein. Dabei kann das Filmscharnier mit verstärkten Enden ausgestattet sein, sodass seine Robustheit gegenüber Querkräften, insbesondere Scherkräften, erhöht wird. Darüber hinaus kann der Verbindungsabschnitt mit mehreren achsparallel, beziehungsweise im Wesentlichen achsparallel, angeordneten Eingelenkscharnieren, beispielweise in Form von mehreren Filmscharnieren, welche sozusagen in Reihe angeordnet sind, ausgebildet sein. Dabei können die mehreren in Reihe angeordneten Filmscharniere an ihren Scharnierenden durch geeignete Mittel miteinander verbunden und hinsichtlich Querkräften verstärkt sein. Auf diese Weise kann die Schwenkbeweglichkeit der Fußorthese weiterhin gesteigert und der Tragekomfort verbessert werden.

Alternativ oder zusätzlich kann die dorsale und/oder plantar anzuordnende Gelenkeinheit ein Biegegelenk umfassen. Die Fußorthese kann derart ausgestaltet sein, dass das Biegegelenk eine geringere Biegesteifigkeit gegenüber Schwenkbewegungen um die Schwenkachse aufweist als daran angrenzende Bereiche des Zehensegments und des Ballensegments.

Hierzu kann das Biegegelenk eine dessen Biegesteifigkeit verringernde, insbesondere dessen Flächenträgheitsmoment gegenüber Biegebelastungen um die Schwenkachse verringernde, geometrische Ausgestaltung aufweisen. Beispielsweise kann die Fußorthese im Bereich des Biegegelenks eine geringere Dicke aufweisen als daran angrenzende Bereiche des Zehensegments und des Ballensegments.

Alternativ oder zusätzlich kann das Biegegelenk eine dessen Biegesteifigkeit verringernde materielle Ausgestaltung aufweisen. Beispielsweise kann die Fußorthese derart ausgestaltet sein, dass sie im Bereich des Biegegelenks ein Material aufweist, das ein geringeres Elastizitätsmodul und/oder eine geringere Festigkeit und/oder eine geringere Härte aufweist als die daran angrenzenden Bereiche.

Alternativ oder zusätzlich kann die Fußorthese im Bereich der Gelenkeinheit ein Verbundmaterial aufweisen, welches Fasern, insbesondere zugstarre Fasern, aufweist, die sich quer zur Schwenkachse beziehungsweise parallel zur Fußorthesen-Längsachse erstrecken. Auf diese Weise kann eine belastungsrechte und langlebige Ausgestaltung der Fußorthese sichergestellt werden.

Gemäß einer Ausführungsform kann das Ballensegment, insbesondere der Ballenbasisabschnitt, mit dem Mittelfußsegment, insbesondere dem Mittelfußbasisabschnitt, kraft- und/oder momentübertragend verbunden sein. Gemäß einer Ausführungsform können das Ballensegment, insbesondere der Ballenbasisabschnitt, und das Mittelfußsegment, insbesondere der Mittelfußbasisabschnitt, derart miteinander verbunden sein, dass das Ballensegment und das Mittelfußsegment relativ zueinander translatorisch verschiebbar sind, insbesondere relativ zu einer Längsachse und/oder einer Querachse der Fußorthese. Hierbei können das Ballensegment und das Mittelfußsegment in einer gewünschten Position relativ zueinander fixierbar sein, insbesondere kraft-und/oder formschlüssig fixierbar sein. Auf diese Weise kann die Fußorthese an die Größe eines zu behandelnden Fußes angepasst werden. Hierfür kann die Kopplung zwischen dem Ballensegment und dem Mittelfußsegment selektiv gesperrt oder freigegeben werden. In einem freigegebenen Zustand der Kopplung zwischen dem Ballensegment und dem Mittelfußsegment, können diese relativ zueinander translatorisch verschoben werden, um eine gewünschte relative Position einzustellen. Daraufhin kann die Kopplung durch Kraft- und/oder Formschluss gesperrt werden, sodass eine relative translatorische Bewegung zwischen dem Ballensegment und dem Mittelfußsegment gesperrt ist.

Alternativ oder zusätzlich kann das Ballensegment mit dem Mittelfußsegment über eine weitere Gelenkeinheit verbunden sein, die insbesondere in einer der vorangehend beschriebenen dorsal und/oder plantar anzuordnenden Gelenkeinheit entsprechenden Art ausgestaltet sein kann.

In einer Weiterentwicklung kann die Fußorthese, insbesondere das Zehensegment und/oder das Ballensegment und/oder das Mittelfußsegment, innenseitig eine Auflage oder Beschichtung, insbesondere eine Polsterbeschichtung, beispielsweise eine polsternde Polyvinylchlorid(PVC)-Beschichtung oder eine Polyurethan(PU)-Beschichtung, aufweisen, wobei die Beschichtung insbesondere weicher ist, d.h. eine geringere Härte aufweist, als das die Beschichtung tragende Teil der Fußorthese. Die Beschichtung kann auf wenigstens einer der Abschnitte oder Segmente der Fußorthese innenseitig aufgebracht sein. Der Begriff "innenseitig" betrifft jene Bereiche der Fußorthese, die im befestigten Zustand dem Fuß zugewandt sind. Die Polsterbeschichtung kann in einem additiven Verfahren als Feststoff oder Gel auf die Innenseiten der Fußorthese beziehungsweise der Schienen aufgebracht werden. Beispielsweise kann die Polsterbeschichtung durch Laminieren oder Kaschieren aufgebracht werden. Weiterhin kann ein geeignetes PVC-Material oder PU-Material aufgeschmolzen und auf die Schiene aufgepresst werden, um die Beschichtung zu erzeugen. Zusätzlich oder alternativ kann ein Auftrag der Beschichtung im flüssigen Zustand erfolgen, wobei der Auftrag mittels Tauchen, Rakeln, Walzenauftrag, Spritzverfahren, Schäumen oder einem anderen geeigneten Verfahren vorgenommen werden kann.

### Kurze Beschreibung der Figuren

Bevorzugte weitere Ausführungsformen der Erfindung werden durch die nachfolgende Beschreibung der Figuren näher erläutert. Dabei zeigen schematisch:
- Figur 1: eine Fußorthese gemäß einer Ausführungsform in einem am Fuß eines Patienten befestigten Zustand in einer perspektivischen Ansicht;
- Figur 2 und 3: die in Figur 1 gezeigte Fußorthese, bei der aus Übersichtsgründen der Fuß nicht dargestellt ist;
- Figur 4 bis 9: die in Figuren 1 und 2 gezeigte Fußorthese in einem vom Fuß entkoppelten Zustand;
- Figur 10: die Fußorthese gemäß einer weiteren Ausführungsform in einer perspektivischen Ansicht;
- Figur 11: die Fußorthese gemäß einer weiteren Ausführungsform in einer perspektivischen Ansicht;
- Figur 12: die Fußorthese gemäß einer weiteren Ausführungsform in einer perspektivischen Ansicht; und
- Figur 13 und 14: die Fußorthese gemäß einer weiteren Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

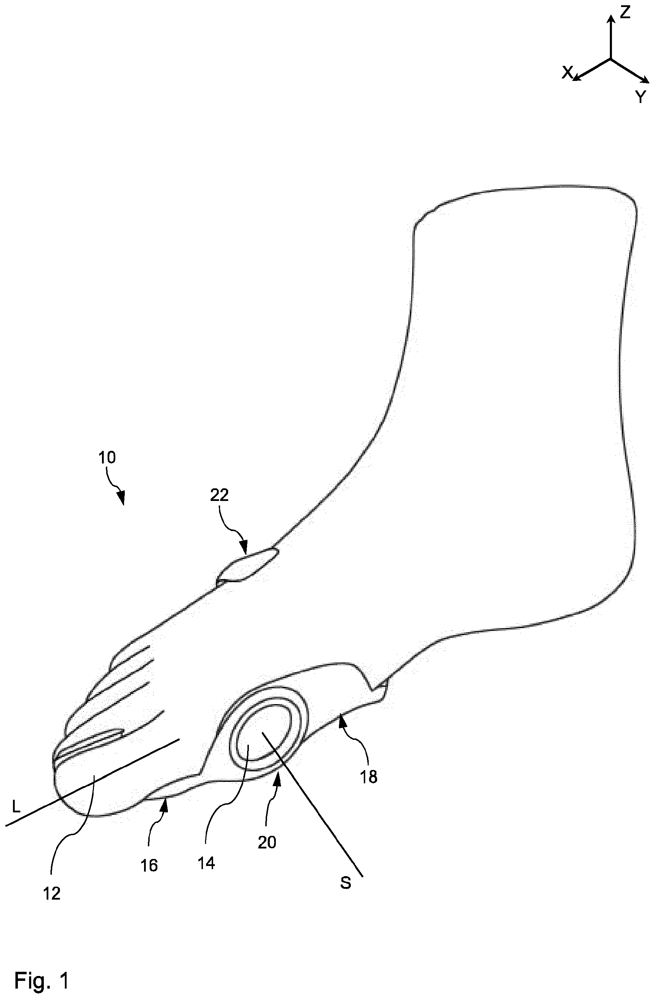

Figur 1 zeigt eine Ausführungsform einer Fußorthese 10 zur Korrektur von Fußfehlstellungen. Genauer ist die in Figur 1 gezeigte Fußorthese dafür vorgesehen, die als Hallux valgus bekannte pathologische Fehlstellung einer Großzehe 12, auch als Valgusstellung der Großzehe bezeichnet, und eines Großzehengrundgelenks 14, auch als Varusstellung des Großzehengrundgelenks bezeichnet, zu behandeln. Auch kann die hier gezeigte Fußorthese 10 zur Vorbeugung von Hallux valgus verwendet werden. Zur Vereinfachung wird nachstehend der Begriff "Großzehe" durch den Begriff "Zehe" abgekürzt und der Begriff "Großzehengrundgelenk" durch "Zehengrundgelenk".

Wie in Figur 1 gezeigt, ist die Fußorthese 10 dafür vorgesehen, an einem zu behandelnden Fuß in der Art einer Klammer, insbesondere Spannklammer, befestigt und in einer vorgegebenen Position fixiert zu werden. Die Fußorthese 10 ist derart ausgebildet und dazu eingerichtet, in dem am Fuß befestigten Zustand therapeutisch auf den Fuß einzuwirken, indem gezielt Korrekturkräfte in den Fuß, insbesondere im Bereich der Zehe 12 und des Zehengrundgelenks 14, eingeleitet werden, wie nachstehend genauer beschrieben wird.

Die gezeigte Fußorthese 10 ist zur Anwendung an dem rechten Fuß des Patienten vorgesehen. Zur Behandlung des linken Fußes des Patienten kann eine zu der in Figur 1 gezeigten Ausgestaltung spiegelsymmetrische Fußorthese eingesetzt werden.

Die Fußorthese 10 umfasst ein an der Zehe 12 befestigbares Zehensegment 16 und ein im Bereich des Zehengrundgelenks 14 angeordnetes und daran befestigbares Ballensegment 18, die mittels einer Gelenkeinheit 20 relativ zueinander verschwenkbar gekoppelt sind. Die Fußorthese 10 umfasst weiterhin ein am oder im Bereich des Mittelfußes befestigbares Mittelfußsegment 22, das kraft- und momentübertragend mit dem Ballensegment 18 verbunden ist.

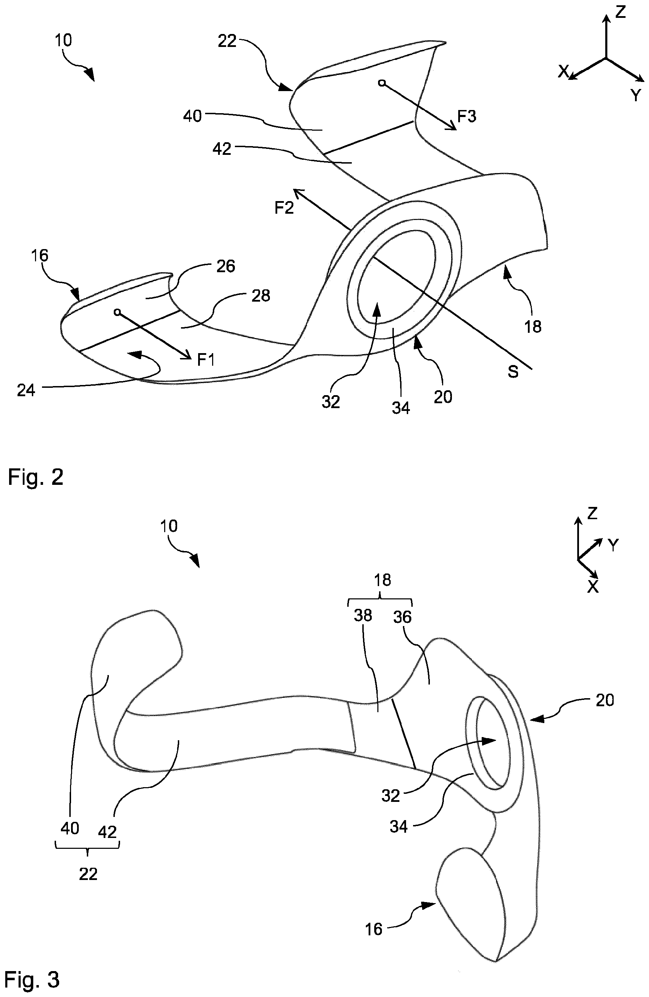

In dem am Fuß befestigten Zustand, wie in Figur 2 angedeutet, ist die Fußorthese 10 dazu eingerichtet, über das Zehensegment 16 eine erste Korrekturkraft F1 auf die Zehe 12 auszuüben, über das Ballensegment 18 eine zu der ersten Korrekturkraft F1 entgegengesetzte und dazu beabstandet parallele zweite Korrekturkraft F2 auf das Zehengrundgelenk 14 auszuüben und über das Mittelfußsegment 22 eine Haltekraft F3, die eine dritte Korrekturkraft bilden kann, auf den Mittelfußbereich auszuüben, wobei die Haltekraft F3 in Richtung der ersten Korrekturkraft F1 zeigt und zu dieser beabstandet parallel angeordnet ist.

Die auf den behandelten Fuß einwirkenden Kräfte F1, F2, F3 bei Gebrauch der Fußorthese 10 sind in Figur 2 dargestellt, in der aus Gründen der besseren Darstellbarkeit der zu behandelnde Fuß nicht gezeigt ist. Zur Vereinfachung werden die entsprechenden Kräfte F1, F2, F3 als einzelne Vektoren dargestellt, wobei im Gebrauch diese selbstverständlich über eine Kontaktfläche zwischen Fuß und Fußorthese verteilt wirken.

Wie nachstehend im Detail beschrieben wird, sind die einzelnen Segmente 16, 18, 22 der Fußorthese 10 elastisch verformbar, wobei die unterschiedlichen auf den Fuß durch die Fußorthese 10 wirkenden Kräfte F1, F2, F3 in Form von durch elastische Verformung der Fußorthese 10 induzierte Spann- und/oder Biegekräfte bereitgestellt werden. Diese Ausgestaltung kann bewirken, dass auch beim Bewegen des Fußes und damit einhergehend bei Veränderung der Form des Fußes im Gebrauch die durch die Fußorthese 10 ausgeübten Kräfte F1, F2, F3 anhaltend auf den Fuß wirken.

Die erste Korrekturkraft F1, die zweite Korrekturkraft F2 und die Haltekraft F3 sind relativ zueinander parallel oder im Wesentlichen parallel und beabstandet zueinander angeordnet. Hierbei zeigen die erste Korrekturkraft F1 und die Haltekraft F3 in mediale Richtung und sind parallel oder im Wesentlichen parallel zu einer Querachse Y der Fußorthese 10. Die zweite Korrekturkraft F2 zeigt in laterale Richtung. Die erste Korrekturkraft F1, die zweite Korrekturkraft F2 und die Haltekraft F3 sind weiterhin orthogonal oder im Wesentlichen orthogonal zu einer Längsachse X und einer Höhenachse Z der Fußorthese 10 angeordnet.

Das Zehensegment 16 ist in Form eines Bügels ausgebildet, insbesondere eines Spann- oder Biegebügels, der in dem am Fuß befestigten Zustand die Zehe 12 umgreift. In der hier gezeigten Ausführungsform erstreckt sich das Zehensegment 16 von einer medialen Seite des Fußes ausgehend von dem Ballensegment 18 über eine Zehenunterseite bis hin zu einer lateralen Seite der Zehe 12. Somit erstreckt sich das Zehensegment 16 abschnittsweise entlang der Zehenunterseite. Alternativ kann sich das Zehensegment 16 auch entlang einer Zehenoberseite erstrecken. In der hier gezeigten Ausführungsform erstreckt sich das Zehensegment 16 über ein Bogenmaß von im Wesentlichen einem π rad um eine Zehenlängsachse L, sodass sich das Zehensegment 16 von einer Seite der Zehe 12 und des Zehengrundgelenks 14 zu der entgegengesetzten Seite der Zehe 12 erstreckt, wie in Figur 1 gezeigt.

Das Zehensegment 16 ist mit einer für die Zehe 12 vorgesehenen Kontaktfläche 24 ausgestaltet. Die Kontaktfläche 24 ist in Form einer Wendefläche ausgestaltet, deren Orientierung, d.h. deren Flächennormale, sich entlang der Zehenlängsachse L ändert und vorzugsweise auf die Zehenlängsachse zeigt. Auf diese Weise erstreckt sich die Kontaktfläche 24 und damit auch das Zehensegment 16 entlang einer Schraubenlinie um die Zehe 12.

Das Zehensegment 16 ist dazu eingerichtet, in dem am Fuß befestigten Zustand Scherkräfte und/oder Biegekräfte in Richtung der ersten Korrekturkraft F1 zwischen der Gelenkeinheit 20 und der zu behandelnden Zehe 12 zu übertragen, um so zur Generierung der ersten Korrekturkraft F1 beizutragen.

Um die erste Korrekturkraft F1 auf die Zehe 12 auszuüben, umfasst das Zehensegment 16 einen Zehenstützabschnitt 26, der in dem befestigten Zustand der Fußorthese 10 an einer lateralen Seite der Zehe 12, d.h. an einer in lateraler Richtung zeigenden Seite der Zehe 12, anliegt. Der Zehenstützabschnitt 26 ist durch einen distalen Endabschnitt des Zehensegments 16 gebildet. Das Zehensegment 16 umfasst ferner einen Zehenbasisabschnitt 28, der mit dem Zehenstützabschnitt 26 integral und stoffschlüssig verbunden ist und an diesen angrenzt, wie durch eine gepunktete Linie in Figur 2 angedeutet.

Das Zehensegment 16, insbesondere der Zehenstützabschnitt 26, ist in der Art einer Biegefeder bereitgestellt. So wird die erste Korrekturkraft F1 in Form einer durch eine elastische Verformung des Zehensegments 16 induzierte Spannkraft oder Biegekraft bereitgestellt. Mit anderen Worten ist das Zehensegment derart ausgestaltet, dass in dem am Fuß befestigten Zustand das Zehensegment 16 in einer Spannposition angeordnet ist, in der das Zehensegment 16 gegenüber einer Ruheposition des Zehensegments 16, in der das Zehensegment 16 in einem vom Fuß entkoppelten Zustand der Fußorthese 10 angeordnet ist, elastisch ausgelenkt und zwar in einer der ersten Korrekturkraft F1 entgegengesetzten Richtung.

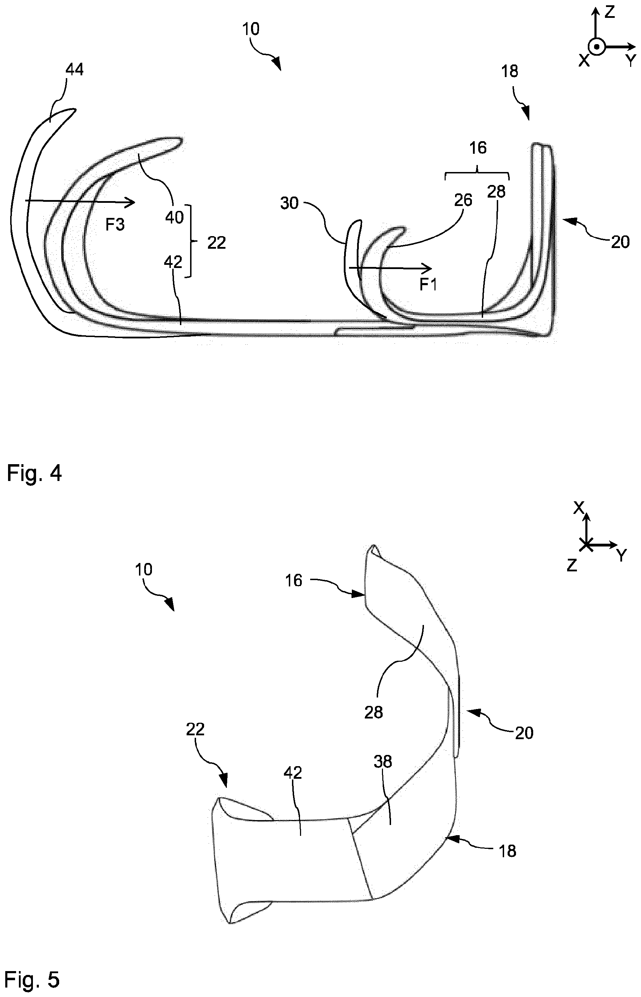

Zur Veranschaulichung dieser Eigenschaft des Zehensegments 16 ist in Fig. 4 die Fußorthese 10 in einem vom Fuß entkoppelten und daher freiliegenden Zustand gezeigt, in dem das Zehensegment 16 in seiner Ruheposition angeordnet ist. Weiterhin ist mithilfe einer gestrichelten Linie 30 ein Zustand des Zehensegments 16 angedeutet, in dem dieses in der Spannposition angeordnet ist, d.h. in dem am Fuß befestigten Zustand. Ein Endabschnitt des Zehenstützabschnitts 26 in der Spannposition ist gegenüber der Ruheposition um wenigstens 0,3 cm oder 0,5 cm, beispielsweise um wenigstens 1,0 cm, ausgelenkt und translatorisch verschoben entlang der zu der ersten Korrekturkraft F1 entgegengesetzten Richtung.

Das Ballensegment 18 ist in dem am Fuß befestigten Zustand der Fußorthese im Bereich des Zehengrundgelenks 14 angeordnet und liegt im Bereich des Zehengrundgelenks 14 an dem Fuß an. Mit anderen Worten ist das Ballensegment 18 dafür vorgesehen, mit dem Fuß im Bereich des Zehengrundgelenks 14 in Eingriff gebracht zu werden, um in dem am Fuß befestigten Zustand der Fußorthese 10 eine kraftübertragende Kopplung zwischen dem Fuß im Bereich des Zehengrundgelenks 14 und dem Ballensegment 18 bereitzustellen. Genauer liegt das Ballensegment 18 in dem am Fuß befestigten Zustand im Bereich einer seitlichen, ballenförmigen Ausbuchtung des Fußes an, die als Pseudoexostose bezeichnet wird. In der hier gezeigten Ausführungsform umfasst das Ballensegment 18 eine Durchgangsöffnung 32, die sich entlang einer Schwenkachse S der Gelenkeinheit 20 erstreckt. Eine die Durchgangsöffnung 32 radial begrenzende Kante 34, insbesondere abgerundete Kante 34, des Ballensegments 18 liegt umlaufend um die ballenförmige Ausbuchtung an dem Fuß an. Mit anderen Worten ist ein Teil des Zehengrundgelenks 14, insbesondere die ballenförmige Ausbuchtung, in der Durchgangsöffnung 32 aufgenommen und angeordnet. Die Durchgangsöffnung 32 an dem Ballensegment 18 kann hierbei im Querschnitt einen Durchmesser, insbesondere einen minimalen Durchmesser, von wenigstens 2 cm oder 2,5 cm aufweisen.

Um die zweite Korrekturkraft F2 auf den Fuß auszuüben, umfasst das Ballensegment 18 einen Ballenstützabschnitt 36, der in dem befestigten Zustand der Fußorthese 10 an dem Grundgelenkfußballen anliegt, im Speziellen medial und plantar an dem Grundgelenkfußballen anliegt. Das Ballensegment 18 kann ferner einen Ballenbasisabschnitt 38 umfassen, der denjenigen Teil des Ballensegments 18 bildet, der sich entlang der Fußsohle des Fußes erstreckt. Der Ballenbasisabschnitt 38 ist mit dem Ballenstützabschnitt 36 integral und stoffschlüssig verbunden und grenzt an diesen an, wie durch eine gepunktete Linie in Figur 3 angedeutet.

Das Ballensegment 18, insbesondere der Ballenstützabschnitt 36, ist in der Art einer Biegefeder bereitgestellt. So wird die zweite Korrekturkraft F2 in Form einer durch eine elastische Verformung des Ballensegments 18 induzierte Spannkraft oder Biegekraft bereitgestellt. Mit anderen Worten ist das Ballensegment 18 derart ausgestaltet, dass in dem am Fuß befestigten Zustand das Ballensegment 18 in einer Spannposition angeordnet ist, in der das Ballensegment gegenüber einer Ruheposition, in der das Ballensegment in einem vom Fuß entkoppelten Zustand der Fußorthese 10 angeordnet ist, elastisch ausgelenkt ist in einer der zweiten Korrekturkraft F2 entgegengesetzten Richtung. Ein Endabschnitt des Ballenstützabschnitts 36 in der Spannposition kann gegenüber der Ruheposition um wenigstens 0,3 cm oder 0,5 cm ausgelenkt und translatorisch verschoben sein entlang der zu der zweiten Korrekturkraft F2 entgegengesetzten Richtung. Die relative Auslenkung des Ballensegments 18 kann geringer sein als diejenige des Zehensegments 16.

Das Ballensegment 18 ist mit dem Mittelfußsegment 22 kraft- und momentübertragend verbunden. Das Mittelfußsegment 22 ist am Mittelfuß des zu behandelnden Fußes befestigbar und teilweise gegenüberliegend zu dem Ballensegment 18, insbesondere dem Ballenstützsegment 36, angeordnet. Das Mittelfußsegment 22 ist in Form eines Bügels, insbesondere Spannbügels, bereitgestellt, der in dem am Fuß befestigten Zustand der Fußorthese 10 einen seitlichen Mittelfußbereich, insbesondere einen lateralen Mittfußbereich, teilweise umgreift. Das als Bügel ausgebildete Mittelfußsegment 22 kann dazu eingerichtet sein, in dem am Fuß befestigten Zustand Scherkräfte und/oder Biegekräfte, insbesondere in Richtung der Haltekraft, aufzunehmen und zwischen dem Ballensegment 18 und dem zu behandelnden Mittelfuß zu übertragen.

Um die Haltekraft in den Mittelfuß einzuleiten, umfasst das Mittelfußsegment 22 einen Mittelfußstützabschnitt 40, der in dem befestigten Zustand der Fußorthese 10 an dem Mittelfuß lateral anliegt. Der Mittelfußstützabschnitt 40 kann durch einen Endabschnitt des Mittelfußsegments 22 gebildet sein. Das Mittelfußsegment 22 umfasst ferner einen Mittelfußbasisabschnitt 42. Der Mittelfußbasisabschnitt 42 ist mit dem Mittelfußstützabschnitt 40 integral und stoffschlüssig verbunden und grenzt an diesen an, wie durch eine gepunktete Linie in Figur 2 angedeutet.

Das Mittelfußsegment 22, insbesondere der Mittelfußstützabschnitt 40 und der Mittelfußbasisabschnitt 42, ist in der Art einer Biegefeder bereitgestellt. So wird die Haltekraft F3 in Form einer durch eine elastische Verformung des Mittelfußsegments 22 induzierte Spannkraft oder Biegekraft bereitgestellt. Mit anderen Worten ist das Mittelfußsegment 22 derart ausgestaltet, dass in dem am Fuß befestigten Zustand das Mittelfußsegment 22 in einer Spannposition angeordnet ist, in der das Mittelfußsegment 22 gegenüber einer Ruheposition des Mittelfußsegments 22, in der das Mittelfußsegment 22 in einem vom Fuß entkoppelten Zustand der Fußorthese 10 angeordnet ist, elastisch ausgelenkt ist in einer der Haltekraft F3 entgegengesetzten Richtung. Diese Eigenschaft ist in Fig. 4 veranschaulicht mithilfe einer weiteren gestrichelten Linie 44, die ein Zustand des Mittelfußsegments 22 angedeutet, in dem dieses in der Spannposition angeordnet ist, d.h. in dem am Fuß befestigten Zustand. Ein Endabschnitt des Mittelfußstützabschnitts 40 in der Spannposition ist gegenüber der Ruheposition um wenigstens 0,5 cm oder 1,0 cm, beispielsweise um 2 cm, ausgelenkt und translatorisch verschoben entlang der zu der Haltekraft F3 entgegengesetzten Richtung.

Zum Fixieren der Fußorthese an dem Fuß kann das Mittelfußsegment 22 und/der das Zehensegment 16 optional mit Bandagen versehen sein. Beispielsweise kann das Zehensegment 16 mittels einer Bandage oder eines Stützbandes die in dem Zehensegment 16 eingespannte Zehe 12 form- und/oder kraftschlüssig relativ zu dem Zehensegment 16 fixieren. Hierzu kann die Bandage oder das Stützband in Umfangsrichtung zumindest abschnittsweise an der Zehe 12 anliegen und mit dem Zehensegment 16 verbunden sein. Weiterhin kann das Mittelfußsegment 22 mit einer zweiten Bandage oder einem zweiten Stützband versehen sein, die um den Mittelfuß in Umfangsrichtung geführt ist und an dessen Enden mit dem Mittelfußsegment 22 verbunden ist, um den Mittelfuß in dem Mittelfußsegment 22 form- und/oder kraftschlüssig zu fixieren.

Das Zehensegment 16 und/oder das Ballensegment 18 und/oder das Mittelfußsegment 22 sind dünnwandig ausgestaltet. Insbesondere kann/können das Zehensegment 16 und/oder das Ballensegment 18 und/oder das Mittelfußsegment 22 aus plattenförmigen und/oder schalenförmigen Elementen aufgebaut sein oder bestehen, die eine maximale Wanddicke von weniger als 3 mm oder 2 mm oder 1 mm aufweisen.

Das Zehensegment 16, das Ballensegment 18 und das Mittelfußsegment 22 sind vorzugsweise aus Kunststoff hergestellt. Insbesondere kommen hierfür Kunststoffe in Frage, die unter hoher Krafteinwirkung, d.h. die höher als die Korrekturkräfte F1, F2 und die Haltekraft F3 sind, oder unter Wärmeeinwirkung plastisch verformbar sind. Auf diese Weise kann die Fußorthese aufwandsreduziert an einen zu behandelnden Fuß in ihrer geometrischen Ausgestaltung angepasst werden. Auch lassen sich so die durch elastische Verformung bewirkten Korrektur- und Haltekräfte F1, F2, F3 anpassen.

Gemäß einer Weiterentwicklung kann wenigstens einer der Stützabschnitte 26, 36, 40 eine geringere Steifigkeit aufweisen, insbesondere gegenüber Scher- und/oder Biegekräfte in Richtung der ersten oder zweiten Korrekturkraft F1, F2, als der daran angrenzende Basisabschnitt 28, 38, 42. Hierzu kann der wenigstens eine Stützabschnitt 26, 36, 40 aus einem Material hergestellt sein, das gegenüber dem Material des angrenzenden Basiselements 28, 38, 42 ein geringeres Elastizitätsmodul oder eine geringere Härte, beispielsweise eine geringer Shore-Härte, aufweist.

Wie vorangehend beschrieben, sind das Zehensegment 16 und das Ballensegment 18 mittels der Gelenkeinheit 20 relativ zueinander verschwenkbar gekoppelt um die Schwenkachse S. In der gezeigten Konfiguration ist die Schwenkachse S der Gelenkeinheit 20 fluchtend oder im Wesentlichen fluchtend zu der Flexion-Extension-Gelenkachse des Zehengrundgelenks 14 angeordnet. Unter der Flexion-Extension-Gelenkachse wird diejenige Gelenkachse verstanden, um die die Zehe 12 bei Flexions- und Extensionsbewegungen verschwenkt wird relativ zum Mittelfuß. Hierzu ist die Gelenkeinheit 20 in dem am Fuß befestigten Zustand an der medialen Seite des Fußes angeordnet.

In der in Figuren 1 bis 9 gezeigten Konfiguration ist die Gelenkeinheit 20 in Form eines Drehgelenks, im Speziellen in Form eines nabenlosen Ringgelenks bereitgestellt, das um die Durchgangsöffnung 32 angeordnet ist. Mit anderen Worten ist die Gelenkeinheit 20 mit der Durchgangsöffnung 32 oder alternativ mit einer Ausnehmung versehen, um den medial ausbuchtenden Teil des Zehengrundgelenks 14, insbesondere die Pseudoexostose, im am Fuß befestigten Zustand teilweise darin aufzunehmen, wie vorangehend im Zusammenhang mit der Durchgangsöffnung 32 bereits beschrieben.

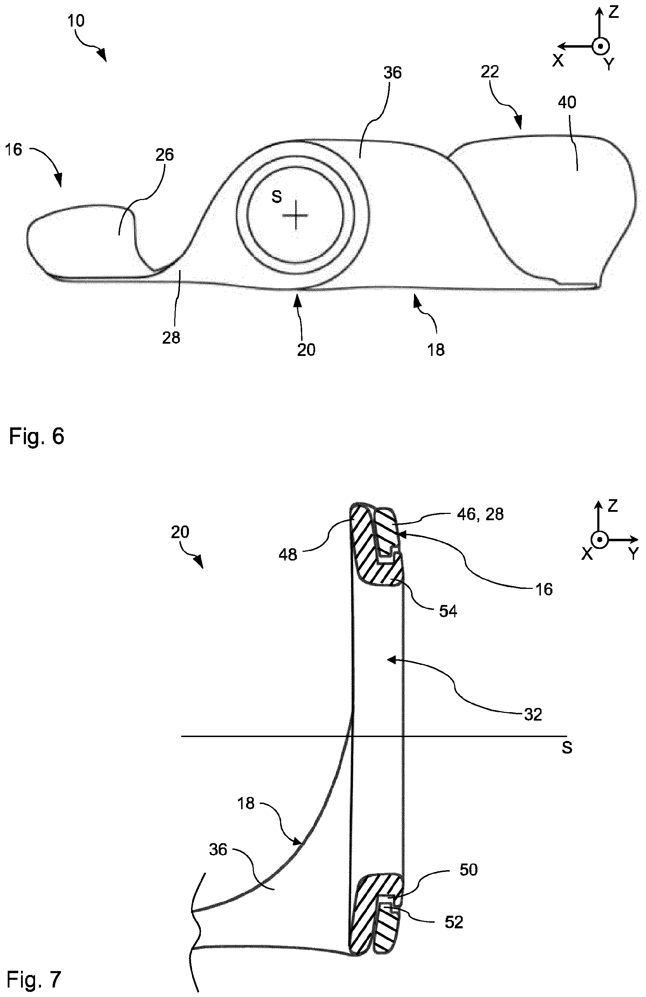

Figur 7 zeigt einen Längsschnitt entlang der Schwenkachse S durch die Gelenkeinheit 20. Die Gelenkeinheit 20 ist aus wenigstens zwei Komponenten gebildet, ist hierauf aber nicht beschränkt und kann in alternativen Ausführungsformen aus mehr als zwei Komponenten gebildet sein.

Genauer umfasst die Gelenkeinheit 20 ein mit dem Zehensegment 16 gekoppeltes, insbesondere integral oder stoffschlüssig damit verbundenes, erstes Gelenkelement 46 und ein dazu komplementär ausgebildetes und im Eingriff stehendes zweites Gelenkelement 48, das mit dem Ballensegment 18 gekoppelt ist, insbesondere integral oder stoffschlüssig damit verbunden ist. Genauer ist das erste Gelenkelement 46 durch einen Endabschnitt des Zehenbasisabschnitts 28 gebildet und das zweite Gelenkelement 48 durch einen Endabschnitt des Ballenstützabschnitts 36. Das zweite Gelenkelement 48 ist mit einer um die Schwenkachse S umlaufenden Aufnahmenut 50 versehen, die mit einem dazu komplementär gestalteten Verbindungsring 52 des ersten Gelenkelements 46 im Eingriff steht. Die Aufnahmenut 50 erstreckt sich in radialer Richtung zu der Schwenkachse S derart, dass die Aufnahmenut 50 in beiden Richtungen entlang der Schwenkachse S begrenzt ist und in der radial nach außen zeigenden Richtung eine Öffnung aufweist, über die der Verbindungsring 52 in die Aufnahmenut 50 ragt. Mit anderen Worten hat die Aufnahmenut 50 im Längsschnitt entlang der Schwenkachse S im Profil eine im Wesentlichen U-förmige Kontakt- oder Gleitfläche für den Verbindungsring 52. So sind die Aufnahmenut 50 und der Verbindungsring 52 in beiden Richtungen entlang der Schwenkachse S formschlüssig im Eingriff. Auf diese Weise kann einem unbeabsichtigten Lösen der Verbindung zwischen dem ersten und dem zweiten Gelenkelement 46, 48 effektiv entgegengewirkt werden und gleichzeitig ein einfacher und robuster Aufbau der Gelenkeinheit 20 sichergestellt werden.

Die Gelenkeinheit 20 ist derart ausgestaltet, dass in dem am Fuß befestigten Zustand der Fußorthese 10, das zweite Gelenkelement 48 zwischen dem Fuß und dem ersten Gelenkelement 46 angeordnet ist, wobei ein Verbindungssteg 54 des zweiten Gelenkelements 48, der die Aufnahmenut 50 ausbildet, die Durchgangsöffnung 32 umlaufend in radialer Richtung begrenzt. Der Verbindungssteg 54 erstreckt sich entlang der Schwenkachse S derart, dass der Verbindungssteg 54 in Axialrichtung der Gelenkeinheit 20, d.h. entlang der Schwenkachse S überlappend zu dem ersten Gelenkelement 46 angeordnet ist und den Verbindungsring 52 umgreift.

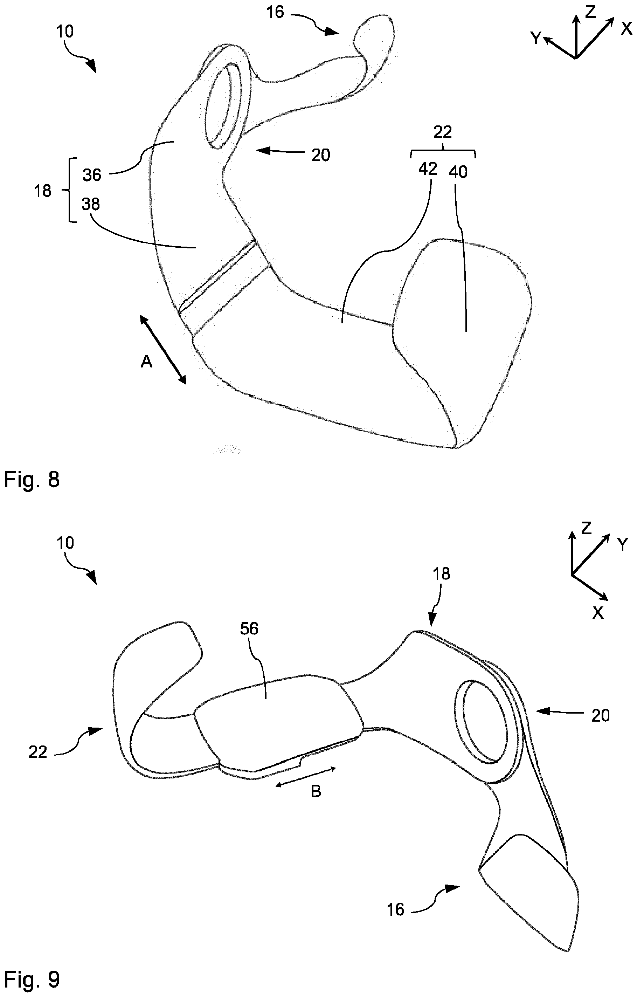

Wie in Figur 8 gezeigt, sind der Ballenbasisabschnitt 38 und der Mittelfußbasisabschnitt 42 derart gekoppelt, dass diese relativ zueinander translatorisch verschiebbar sind entlang einer Achse, die in einer Ebene mit der Längsachse X und einer Querachse Y der Fußorthese 10 liegt, aber quer zu diesen verläuft, wie in Figur 8 durch den Pfeil A angedeutet. Mit anderen Worten sind die beiden Abschnitte 38, 42 relativ zueinander translatorisch verschiebbar entlang der Längsachse X und der Querachse Y der Fußorthese 10. Die Fußorthese 10 ist hierbei derart ausgestaltet, dass die beiden Abschnitte 38, 42 in einer gewünschten Position relativ zueinander kraft- und/oder formschlüssig fixierbar sind.

Optional kann die Fußorthese 10 ferner ein Fußkissen 56 umfassen, das lösbar mit dem Ballenbasisabschnitt 38 und/oder dem Mittelfußbasisabschnitt 42 verbunden werden kann und relativ zu diesen verschiebbar ist, um das Fußkissen 56 patientenspezifisch anzuordnen, wie in Figur 9 durch den Pfeil B angedeutet.

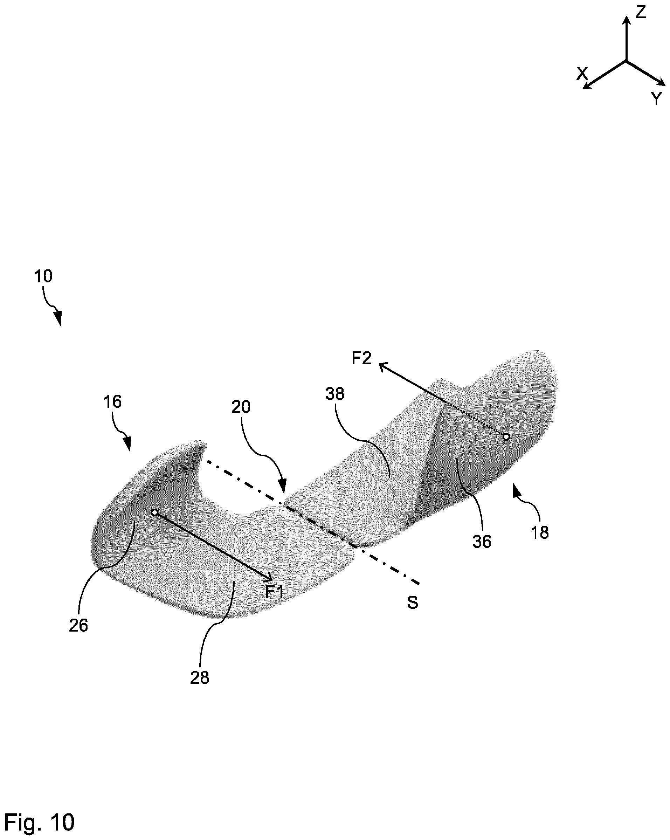

Figur 10 zeigt eine zweite Ausführungsform der Fußorthese 20, die sich insbesondere durch den Aufbau und die Anordnung des Zehensegments 16, des Ballensegments 18 und der Gelenkeinheit 20 von der in Figuren 1 bis 9 gezeigten Ausführungsform unterscheidet.

In der in Figur 10 gezeigten Ausführungsform ist die Gelenkeinheit 20 entlang der Fußsohle in dem am Fuß befestigten Zustand angeordnet. Mit anderen Worten ist die Gelenkeinheit 20 plantar, d.h. fußsohlenseitig oder im Bereich der Fußsohle, am Fuß im Gebrauch angeordnet. Die Gelenkeinheit 20 ist als Biegegelenk bereitgestellt, das durch Endabschnitte des platten- oder schalenförmigen Zehenbasisabschnitts 28 und des Ballenbasisabschnitts 38 ausgebildet ist. Vorzugsweise erstreckt sich die Gelenkeinheit 20 über eine Länge entlang der Querachse Y von wenigstens 1 cm. In der gezeigten Ausführungsform erstreckt sich die Gelenkeinheit 20 entlang der Querachse Y im Wesentlichen über die gesamte Breite der miteinander in Eingriff stehenden Endabschnitte des Zehenbasisabschnitts 28 und des Ballenbasisabschnitts 38.

Der Zehenbasisabschnitt 28 und der Ballenbasisabschnitt 38 erstrecken sich platten- oder schalenförmig entlang einer Ebene, die parallel zu den Korrekturkräften F1, F2 verläuft. In dieser gedachten Ebene liegt und erstreckt sich auch die Gelenkeinheit 20. Auf diese Weise ist die vorgeschlagenen Fußorthese 10, insbesondere die Gelenkeinheit 20, besonders robust ausgestaltet gegenüber Biege- und Scherkräften in Richtung der Korrekturkräfte F1, F2.

Die als Biegegelenk bereitgestellte Gelenkeinheit 20 hat eine geringere Biegesteifigkeit gegenüber Schwenkbewegungen um die Schwenkachse S als daran angrenzende Bereiche des Zehensegments 16 und des Ballensegments 18. Dies kann dadurch erreicht werden, dass die Fußorthese 10 im Bereich der Gelenkeinheit 20 dünner und/oder aus einem gegenüber angrenzenden Bereichen biegeweicherem Material hergestellt ist. Weiterhin kann die Fußorthese im Bereich der Gelenkeinheit 20 ein Verbundmaterial aufweisen, welches Fasern, insbesondere zugstarre Fasern, aufweist, die sich quer zur Schwenkachse S beziehungsweise parallel zur Fußorthesen-Längsachse X erstrecken. Insbesondere kann die Gelenkeinheit 20 als Filmscharnier ausgebildet sein. Alternativ kann die Gelenkeinheit 20 auch als Drehgelenk, insbesondere als Eingelenkscharnier ausgebildet sein.

Die hier gezeigte Fußorthese 10 kommt ohne ein Mittelfußsegment aus. Um diese dennoch in einer stabilen Position am zu behandelnden Fuß zu halten, die das Aufbringen der Korrekturkräfte ermöglicht, kann die hier gezeigte Ausführungsform dafür vorgesehen sein, in Kombination mit einem Strumpf oder Schuhwerk getragen zu werden, wodurch die für die therapeutische Behandlung hilfreichen, die Fußorthese 10 am Fuß haltenden Haltekräfte bereitgestellt werden.

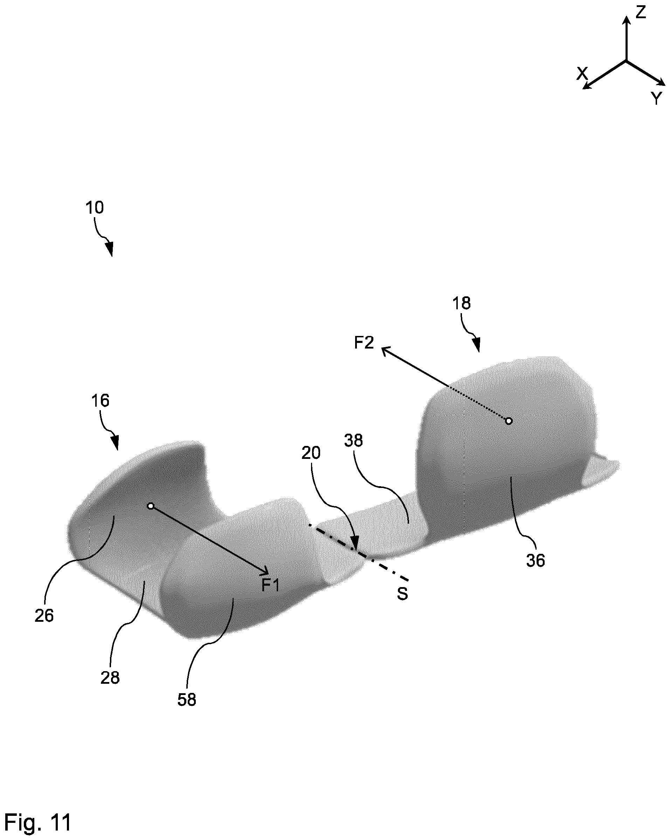

Figur 11 zeigt eine weitere Ausführungsform der Fußorthese 10, bei der das Zehensegment 16 gegenüber der in Figur 10 gezeigten Ausführungsform mit einem dem Zehenstützabschnitt 26 gegenüberliegenden medialen Seitenabschnitt 58 versehen ist.

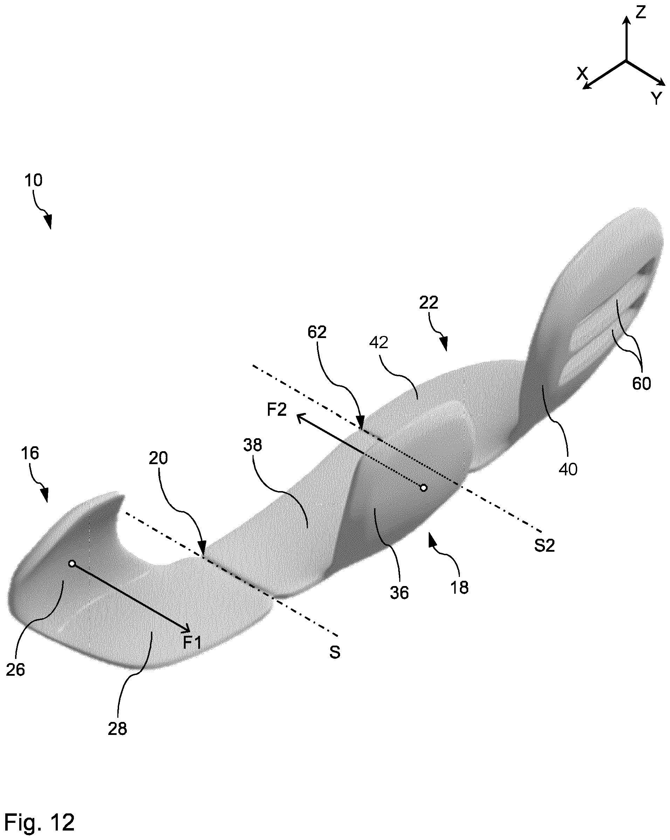

Figur 12 zeigt eine weitere Ausführungsform der Fußorthese 10, die gegenüber den in Figuren 10 und 11 gezeigten Ausführungsformen mit einem Mittelfußsegment 22 ausgestattet ist. Das Mittelfußsegment 22 umfasst einen Mittelfußstützabschnitt 40, der mit Kopplungselementen 60 für ein hier nicht gezeigtes Stützband versehen ist. Die Kopplungselemente 60 sind in der hier gezeigten Ausführungsform als Schlitzöffnungen bereitgestellt, durch welche das Stützband beim Umschlingen des Mittelfußes geführt wird, um so die Fußorthese 10 am zu behandelnden Fuß zu fixieren und entsprechend die Haltekraft über das Stützband in den Fuß einzuleiten. Das Mittelfußsegment 22 ist mit dem Ballensegment 18 über eine weitere Gelenkeinheit 62 schwenkbar um eine weitere Schwenkachse S2 gekoppelt, die parallel zu der Schwenkachse S der Gelenkeinheit 20 zwischen dem Zehensegment 16 und dem Ballensegment 18 ausgebildet ist. Die weitere Gelenkeinheit 62 ist entsprechend zu der Gelenkeinheit 20 ausgestaltet.

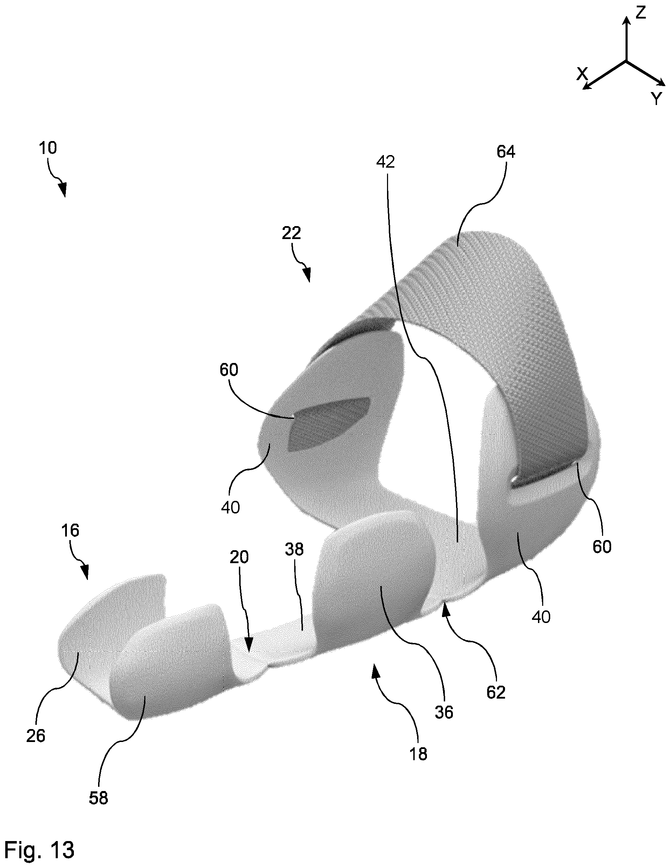

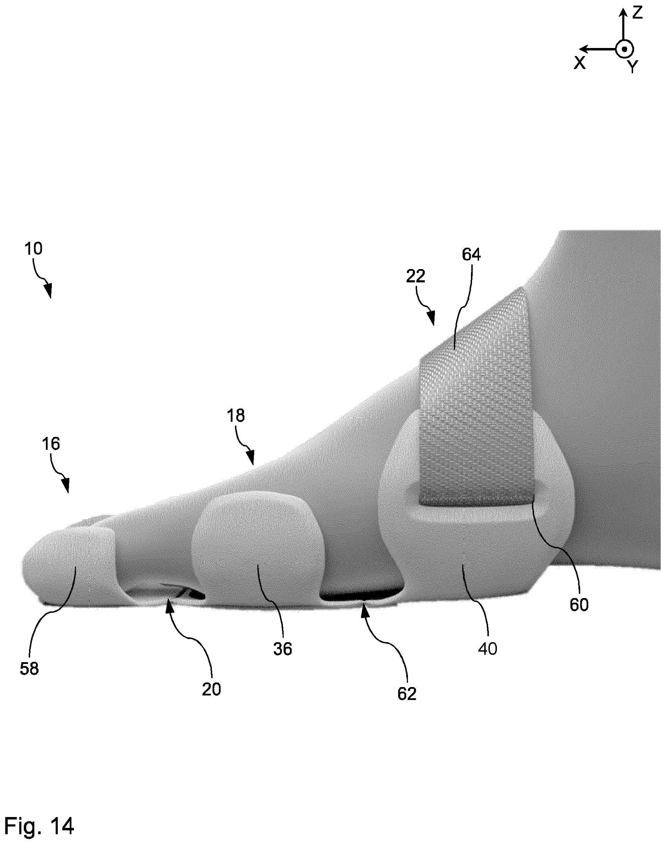

Figuren 13 und 14 zeigen eine weitere Ausführungsform der Fußorthese 10, bei der das Mittelfußsegment 22 zwei gegenüberliegende Mittelfußstützabschnitte 40 umfasst mit jeweils einem Kopplungselement 60, durch die ein den Mittelfuß zu umgreifendes Stützband 64 geführt ist.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 10: Fußorthese
- 12: Großzehe
- 14: Großzehengrundgelenk
- 16: Zehensegment
- 18: Ballensegment
- 20: Gelenkeinheit
- 22: Mittelfußsegment
- 24: Kontaktfläche
- 26: Zehenstützabschnitt
- 28: Zehenbasisabschnitt
- 30: Spannposition des Zehensegments
- 32: Ausnehmung oder Durchgangsöffnung
- 34: Kante der Durchgangsöffnung
- 36: Ballenstützabschnitt
- 38: Ballenbasisabschnitt
- 40: Mittelfußstützabschnitt
- 42: Mittelfußbasisabschnitt
- 44: Spannposition des Mittelfußsegments
- 46: erstes Gelenkelement
- 48: zweites Gelenkelement
- 50: Aufnahmenut
- 52: Verbindungsring
- 54: Verbindungssteg
- 56: Fußkissen
- 58: Seitenabschnitt
- 60: Kopplungselement
- 62: weitere Gelenkeinheit
- 64: Stützband
- F1: erste Korrekturkraft
- F2: zweite Korrekturkraft
- F3: Haltekraft
- L: Zehenlängsachse
- S: Schwenkachse
- S2: weitere Schwenkachse

## Patentansprüche

1. Fußorthese (10) zur Korrektur von Fußfehlstellungen, insbesondere zur Behandlung von Hallux valgus, umfassend ein an einer Zehe (12) befestigbares Zehensegment (16) und ein im Bereich eines Zehengrundgelenks (14) anzuordnendes Ballensegment (18), die mittels einer Gelenkeinheit (20) relativ zueinander verschwenkbar gekoppelt sind, wobei
die Fußorthese (10) dazu eingerichtet ist, in einem am Fuß ordnungsgemäß befestigten Zustand über das Zehensegment (16) eine erste Korrekturkraft (F1) auf die Zehe (12) auszuüben und über das Ballensegment (18) eine zu der ersten Korrekturkraft (F1) entgegengesetzte zweite Korrekturkraft (F2) auf das Zehengrundgelenk (14) auszuüben, wobei
die Gelenkeinheit (20) derart ausgebildet ist, dass das Zehensegment (16) und das Ballensegment (18) relativ zueinander um eine Schwenkachse (S) verschwenkbar sind, die im Wesentlichen parallel zu einer Flexion-Extension-Gelenkachse des Zehengrundgelenks (14) ist, wobei
das Zehensegment (16) in Form eines Bügels ausgebildet ist, der in dem am Fuß befestigten Zustand die Zehe (12) zumindest teilweise umgreift, und wobei das Zehensegment (16) einen Zehenstützabschnitt (26) umfasst, der in dem am Fuß befestigten Zustand der Fußorthese (10) an einer lateralen Seite der Zehe anliegt.

2. Fußorthese nach Anspruch 1, die derart ausgebildet ist, dass sich das Zehensegment (16) in dem am Fuß befestigten Zustand abschnittsweise entlang einer Zehenunterseite oder entlang einer Zehenoberseite der Zehe (12) erstreckt.

3. Fußorthese nach Anspruch 1 oder 2, wobei das Zehensegment (16) dazu eingerichtet ist, in dem am Fuß befestigen Zustand Scherkräfte und/oder Biegekräfte in Richtung der ersten Korrekturkraft (F1) zwischen der Gelenkeinheit (20) und der zu behandelnden Zehe (12) zu übertragen, um die erste Korrekturkraft (F1) zu generieren.

4. Fußorthese nach einem der Ansprüche 1 bis 3, wobei die erste Korrekturkraft (F1) in Form einer durch eine elastische Verformung des Zehensegments (16) induzierte Spannkraft oder Biegekraft bereitgestellt ist.

5. Fußorthese nach einem der Ansprüche 1 bis 4, wobei das Zehensegment (16) in dem am Fuß befestigten Zustand in einer Spannposition angeordnet ist, in der das Zehensegment (16) gegenüber einer Ruheposition des Zehensegments (16), in der das Zehensegment (16) in einem vom Fuß entkoppelten Zustand der Fußorthese (10) angeordnet ist, elastisch ausgelenkt ist in einer der ersten Korrekturkraft (F1) entgegengesetzten Richtung.

6. Fußorthese nach einem der Ansprüche 1 bis 5, die derart ausgestaltet ist, dass in dem am Fuß befestigten Zustand der Fußorthese (10) ein seitlich ausbuchtender Teil eines Grundgelenkfußballens in einer Ausnehmung oder Durchgangsöffnung (32) des Ballensegments (18) aufgenommen ist.

7. Fußorthese nach einem der Ansprüche 1 bis 6, wobei die zweite Korrekturkraft (F2) in Form einer durch eine elastische Verformung des Ballensegments (18) induzierte Spannkraft bereitgestellt ist.

8. Fußorthese nach einem der Ansprüche 1 bis 7, die ferner ein am Mittelfuß des zu behandelnden Fußes befestigbares Mittelfußsegment (22) umfasst, das in dem am Fuß befestigten Zustand eine Haltekraft (F3) auf den Mittelfuß ausübt, und/oder
die ein an der Ferse des zu behandelnden Fußes befestigbares Fersensegment umfasst, das in dem am Fuß befestigten Zustand eine Haltekraft auf einen hinteren Teil des Fußes ausübt.

9. Fußorthese nach einem der Ansprüche 1 bis 8, wobei das Zehensegment (16) und/oder das Ballensegment (18) und/oder das Mittefußsegment (22) einen Basisabschnitt (28, 38, 42) und einen Korrektur- oder Haltekräfte in den Fuß einleitenden Stützabschnitt (26, 36, 40) aufweisen, wobei der Stützabschnitt (26, 36, 40) gegenüber dem Basisabschnitt (28, 38, 42) eine geringere Steifigkeit aufweist gegenüber Scher- und/oder Biegekräften in Richtung der ersten oder zweiten Korrekturkraft (F1, F2).

10. Fußorthese nach Anspruch 9, wobei der Stützabschnitt (26, 36, 40) aus einem Material hergestellt ist, das ein geringeres Elastizitätsmodul oder eine geringere Festigkeit oder eine geringere Härte aufweist als der Basisabschnitt (28, 38, 42).

11. Fußorthese nach einem der Ansprüche 1 bis 10, wobei die Gelenkeinheit (20) in dem am Fuß befestigten Zustand seitlich am Fuß angeordnet ist oder sich entlang des Fußrückens oder entlang der Fußsohle erstreckt, und wobei
die Gelenkeinheit (20) in Form eines Drehgelenks oder eines Biegegelenks bereitgestellt ist.

12. Fußorthese nach einem der Ansprüche 1 bis 11, wobei die Gelenkeinheit (20) in Form eines nabenlosen Drehgelenks bereitgestellt ist, das entlang seiner Schwenkachse (S) mit einer Ausnehmung oder einer Durchgangsöffnung (32) versehen ist.

13. Fußorthese nach einem der Ansprüche 6 bis 12, wobei die Ausnehmung oder Durchgangsöffnung (32) einen Durchmesser von wenigstens 2,0 cm oder 2,5 cm aufweist.

14. Fußorthese nach einem der Ansprüche 1 bis 13, wobei das Ballensegment (18) und das Mittelfußsegment (22) derart miteinander gekoppelt sind, dass diese relativ zueinander translatorisch verschiebbar sind, insbesondere entlang einer Längsachse (X) und/oder einer Querachse (Y) der Fußorthese (10), und in einer gewünschten Position relativ zueinander kraft- und/oder formschlüssig fixierbar sind.

## Claims

1. Foot orthosis (10) for correcting foot malpositions, in particular for the treatment of hallux valgus, comprising a toe segment (16) that can be fastened to a toe (12) and a ball-of-foot segment (18) to be arranged in the region of a metatarsophalangeal joint (14), which segments are pivotably connected relative to one another by means of a joint unit (20), wherein
when correctly fastened to the foot, the foot orthosis (10) is configured to exert a first corrective force (F1) on the toe (12) via the toe segment (16) and to exert a second corrective force (F2) opposite to the first corrective force (F1) on the metatarsophalangeal joint (14) via the ball-of-foot segment (18), wherein
the joint unit (20) is configured such that the toe segment (16) and the ball-of-foot segment (18) are pivotable relative to each other about a pivot axis (S) which is substantially parallel to a flexion-extension joint axis of the metatarsophalangeal joint (14), wherein
wherein the toe segment (16) is provided in the form of a bracket which, when fastened to the foot, at least partially engages around the toe (12), and wherein the toe segment (16) comprises a toe support section (26) which, when the foot orthosis (10) is fastened to the foot, lies against a lateral side of the toe.

2. Foot orthosis according to claim 1, which is configured such that, when fastened to the foot, the toe segment (16) extends in sections along a lower side or along an upper side of the toe (12).

3. Foot orthosis according to claim 1 or 2, wherein, when fastened to the foot, the toe segment (16) is configured to transmit shear forces and/or bending forces in the direction of the first corrective force (F1) between the joint unit (20) and the toe (12) to be treated in order to generate the first corrective force (F1).

4. Foot orthosis according to any one of claims 1 to 3, wherein the first corrective force (F1) is provided in the form of a clamping force or bending force induced by elastic deformation of the toe segment (16).

5. Foot orthosis according to any one of claims 1 to 4, wherein, when fastened to the foot, the toe segment (16) is arranged in a clamping position in which the toe segment (16) is elastically deflected in a direction opposite to the first corrective force (F1) relative to a rest position of the toe segment (16) in which the toe segment (16) is arranged in a state in which the foot orthosis (10) is decoupled from the foot.

6. Foot orthosis according to any one of claims 1 to 5, which is configured such that, when the foot orthosis (10) is fastened to the foot, a laterally protruding part of a metatarsophalangeal ball is received in a recess or through-hole (32) of the ball-of-foot segment (18).

7. Foot orthosis according to any one of claims 1 to 6, wherein the second corrective force (F2) is provided in the form of a clamping force induced by elastic deformation of the ball-of-foot segment (18).

8. Foot orthosis according to any one of claims 1 to 7, further comprising a metatarsal segment (22) which is to be fastened to the metatarsus of the foot to be treated and which, when fastened to the foot, exerts a holding force (F3) on the metatarsus, and/or
comprising a heel segment which is to be fastened to the heel of the foot to be treated, and which, when fastened to the foot, exerts a holding force on a rear part of the foot.

9. Foot orthosis according to any one of claims 1 to 8, wherein the toe segment (16) and/or the ball-of-foot segment (18) and/or the metatarsal segment (22) have a base section (28, 38, 42) and a support section (26, 36, 40) introducing corrective or holding forces into the foot, wherein compared to the base section (28, 38, 42), the support section (26, 36, 40) has a lower stiffness against shear and/or bending forces in the direction of the first or second corrective force (F1, F2).

10. Foot orthosis according to claim 9, wherein the support section (26, 36, 40) is made of a material having a lower modulus of elasticity or a lower strength or a lower hardness than the base section (28, 38, 42).

11. Foot orthosis according to any one of claims 1 to 10, wherein, when fastened to the foot, the joint unit (20) is arranged laterally at the foot or extends along the dorsum of the foot or along the sole of the foot, and wherein
the joint unit (20) is provided in the form of a swivel joint or a bending joint.

12. Foot orthosis according to any one of claims 1 to 11, wherein the joint unit (20) is provided in the form of a hubless swivel joint which is provided with a recess or a through-hole (32) along its pivot axis (5).

13. Foot orthosis according to any one of claims 6 to 12, wherein the recess or through-hole (32) has a diameter of at least 2.0 cm or 2.5 cm.

14. Foot orthosis according to any one of claims 1 to 13, wherein the ball-of-foot segment (18) and the metatarsal segment (22) are coupled to each other such that they can be displaced translationally relative to each other, in particular along a longitudinal axis (X) and/or a transverse axis (Y) of the foot orthosis (10), and are fixable in a desired position relative to each other in a force-fitting and/or form-fitting manner.

## Revendications

1. Orthèse de pied (10) pour la correction de malformations du pied, en particulier pour le traitement de l'hallux valgus, comprenant un segment d'orteil (16) pouvant être fixé à un orteil (12) et un segment de coussin plantaire (18) à disposer au niveau d'une articulation de base d'orteil (14), qui sont couplés de manière pivotante l'un par rapport à l'autre au moyen d'une unité d'articulation (20), dans laquelle
l'orthèse de pied (10) est configurée pour exercer, dans un état correctement fixé au pied, une première force de correction (F1) sur l'orteil (12) par l'intermédiaire du segment d'orteil (16) et une seconde force de correction (F2) opposée à la première force de correction (F1) sur l'articulation de base d'orteil (14) par l'intermédiaire du segment de coussin plantaire (18), dans laquelle
l'unité d'articulation (20) est conçue de sorte que le segment d'orteil (16) et le segment de coussin plantaire (18) peuvent pivoter l'un par rapport à l'autre autour d'un axe de pivotement (S) qui est sensiblement parallèle à un axe d'articulation de flexion-extension de l'articulation de base d'orteil (14), dans laquelle
le segment d'orteil (16) est conçu sous forme d'un étrier qui, dans l'état fixé au pied, entoure au moins partiellement l'orteil (12), et dans laquelle le segment d'orteil (16) comprend une section de support d'orteil (26) qui, dans l'état fixé au pied de l'orthèse de pied (10), repose contre un côté latéral de l'orteil.

2. Orthèse de pied selon la revendication 1, qui est conçue de sorte que, dans l'état fixé au pied, le segment d'orteil (16) s'étend par sections le long d'un côté inférieur d'orteil ou le long d'un côté supérieur d'orteil de l'orteil (12).

3. Orthèse de pied selon la revendication 1 ou 2, dans laquelle le segment d'orteil (16) est configuré, dans l'état fixé au pied, pour transmettre des forces de cisaillement et/ou des forces de flexion en direction de la première force de correction (F1) entre l'unité d'articulation (20) et l'orteil (12) à traiter, pour générer la première force de correction (F1).

4. Orthèse de pied selon l'une quelconque des revendications 1 à 3, dans laquelle la première force de correction (F1) est fournie sous forme d'une force de tension ou d'une force de flexion induite par une déformation élastique du segment d'orteil (16).

5. Orthèse de pied selon l'une quelconque des revendications 1 à 4, dans laquelle le segment d'orteil (16) est disposé, dans l'état fixé au pied, dans une position de serrage dans laquelle le segment d'orteil (16) est dévié élastiquement dans une direction opposée à la première force de correction (F1) par rapport à une position de repos du segment d'orteil (16) dans laquelle le segment d'orteil (16) est disposé dans un état découplé du pied de l'orthèse de pied (10).

6. Orthèse de pied selon l'une quelconque des revendications 1 à 5, qui est conçue de sorte que, dans l'état fixé au pied de l'orthèse de pied (10), une partie latéralement courbée d'un coussin plantaire d'articulation de base est logée dans un évidement ou une ouverture traversante (32) du segment de coussin plantaire (18).

7. Orthèse de pied selon l'une quelconque des revendications 1 à 6, dans laquelle la seconde force de correction (F2) est fournie sous forme d'une force de tension induite par une déformation élastique du segment de coussin plantaire (18).

8. Orthèse de pied selon l'une quelconque des revendications 1 à 7, qui comprend en outre un segment métatarsien (22) pouvant être fixé au métatarse du pied à traiter, qui exerce une force de maintien (F3) sur le métatarse dans l'état fixé au pied, et/ou
qui comprend un segment de talon pouvant être fixé au talon du pied à traiter et qui, dans l'état fixé au pied, exerce une force de maintien sur une partie arrière du pied.

9. Orthèse de pied selon l'une quelconque des revendications 1 à 8, dans laquelle le segment d'orteil (16) et/ou le segment de coussin plantaire (18) et/ou le segment de métatarse (22) présentent une section de base (28, 38, 42) et une section de support (26, 36, 40) introduisant des forces de correction ou de maintien dans le pied, dans laquelle la section de support (26, 36, 40) présente, par rapport à la section de base (28, 38, 42), une rigidité moindre par rapport aux forces de cisaillement et/ou de flexion en direction de la première ou de la seconde force de correction (F1, F2).

10. Orthèse de pied selon la revendication 9, dans laquelle la partie de support (26, 36, 40) est fabriquée dans un matériau qui présente un module d'élasticité inférieur ou une résistance inférieure ou une dureté inférieure à celle de la section de base (28, 38, 42).

11. Orthèse de pied selon l'une quelconque des revendications 1 à 10, dans laquelle l'unité d'articulation (20), dans l'état fixé au pied, est disposée latéralement au pied ou s'étend le long du dos du pied ou le long de la plante du pied, et dans laquelle
l'unité d'articulation (20) est fournie sous forme d'un pivot ou d'une articulation de flexion.

12. Orthèse de pied selon l'une quelconque des revendications 1 à 11, dans laquelle l'unité d'articulation (20) est fournie sous forme d'un pivot sans moyeu, qui est doté d'un évidement ou d'une ouverture traversante (32) le long de son axe de pivotement (S).

13. Orthèse de pied selon l'une quelconque des revendications 6 à 12, dans laquelle l'évidement ou l'ouverture traversante (32) présente un diamètre d'au moins 2,0 cm ou 2,5 cm.

14. Orthèse de pied selon l'une quelconque des revendications 1 à 13, dans laquelle le segment de coussin plantaire (18) et le segment métatarsien (22) sont couplés l'un à l'autre de sorte que ceux-ci peuvent être déplacés en translation l'un par rapport à l'autre, en particulier le long d'un axe longitudinal (X) et/ou d'un axe transversal (Y) de l'orthèse de pied (10), et peuvent être fixés l'un par rapport à l'autre par complémentarité de force et/ou de forme dans une position souhaitée.
